(19) Europäisches Patentamt
European Patent Office
Office européen des brevets

(11) **EP 3 046 668 B1**

(12) **EUROPÄISCHE PATENTSCHRIFT**

(45) Veröffentlichungstag und Bekanntmachung des Hinweises auf die Patenterteilung:
**12.06.2019 Patentblatt 2019/24**

(21) Anmeldenummer: **14766463.5**

(22) Anmeldetag: **15.09.2014**

(51) Int Cl.:
*B01J 23/887* [(2006.01)]    *B01J 23/888* [(2006.01)]
*B01J 23/94* [(2006.01)]    *B01J 35/00* [(2006.01)]
*B01J 35/10* [(2006.01)]    *B01J 37/02* [(2006.01)]
*C07C 51/235* [(2006.01)]    *B01J 35/02* [(2006.01)]
*B01J 35/04* [(2006.01)]    *B01J 37/04* [(2006.01)]
*B01J 37/08* [(2006.01)]    *C07C 51/25* [(2006.01)]

(86) Internationale Anmeldenummer:
**PCT/EP2014/069580**

(87) Internationale Veröffentlichungsnummer:
**WO 2015/039982 (26.03.2015 Gazette 2015/12)**

(54) **KATALYSATOR ZUR HERSTELLUNG EINER UNGESÄTTIGTEN CARBONSÄURE DURCH GASPHASENOXIDATION EINES UNGESÄTTIGTEN ALDEHYDS**

CATALYST FOR PRODUCING AN UNSATURATED CARBOXYLIC ACID BY GAS PHASE OXIDATION OF AN UNSATURATED ALDEHYDE

CATALYSEUR POUR LA PRÉPARATION D'UN ACIDE CARBOXYLIQUE INSATURÉ PAR OXYDATION EN PHASE GAZEUSE D'UN ALDÉHYDE INSATURÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.09.2013 DE 102013218628**
**17.09.2013 US 201361878651 P**

(43) Veröffentlichungstag der Anmeldung:
**27.07.2016 Patentblatt 2016/30**

(73) Patentinhaber: **BASF SE**
**67056 Ludwigshafen am Rhein (DE)**

(72) Erfinder:
• **WELKER-NIEUWOUDT, Cathrin Alexandra**
**67134 Birkenheide (DE)**
• **DOBNER, Cornelia Katharina**
**67071 Ludwigshafen (DE)**
• **BORCHERT, Holger**
**67591 Offstein (DE)**
• **HAMMON, Ulrich**
**68163 Mannheim (DE)**
• **MACHT, Josef**
**B-2000Z Antwerpen 1 (BE)**
• **KARPOV, Andrey**
**Cranford, New Jersey 07016 (US)**
• **WALSDORFF, Christian**
**67061 Ludwigshafen (DE)**

(56) Entgegenhaltungen:
WO-A1-2004/085370    WO-A2-2005/030380
DE-A1- 10 028 582    US-A1- 2004 015 013

<u>Bemerkungen:</u>
Die Akte enthält technische Angaben, die nach dem Eingang der Anmeldung eingereicht wurden und die nicht in dieser Patentschrift enthalten sind.

**Beschreibung**

Katalysatoren aus einem Trägerformkörper sowie einer auf die äußere Oberfläche des Trägerkörpers aufgebrachten, wenigstens die Elemente Mo, V und Cu enthaltenden, Schale aus katalytisch aktiver Oxidmasse sind bekannt (vgl. z.B. EP-A 714 700, DE-A 199 27 624, DE-A 10360057, WO 2011/134932 A1 und DE-A 10028582). Sie werden hauptsächlich als Katalysatoren für die heterogen katalysierte partielle Gasphasenoxidation von Acrolein zu Acrylsäure eingesetzt. Die WO 2005/030380 A2 beschreibt ein Verfahren zur Herstellung von Trägerkatalysatoren, bei dem man Katalysatorträger in einer Vorrichtung, die mit einer haftvermindernden Beschichtung versehen ist, mit einer katalysatorhaltigen Suspension beschichtet.

Diese Katalysatoren weisen jedoch Nachteile auf. Bei der Verwendung als Katalysatoren für die heterogen katalysierte partielle Gasphasenoxidation von Acrolein zu Acrylsäure ist die Selektivität der Acrylsäurebildung nicht in vollem Umfang zufriedenstellend. Als Nebenreaktion tritt insbesondere die Überoxidation zu CO und $CO_2$ (im Folgenden zusammen als $CO_x$ bezeichnet) auf.

Ein ausreichend hoher Umsatz an Acrolein wird bei Verwendung der Katalysatoren des Stands der Technik oft nur unter Bedingungen erzielt, unter denen die Selektivität der Acrylsäurebildung nicht zufriedenstellend ist. So kommt es bei den Temperaturen, unter denen ein ausreichend hoher Umsatz an Acrolein erreicht wird, häufig zur Überoxidation und folglich zu einer Verringerung der Selektivität der Acrylsäurebildung.

Die WO 2004/085370 beschreibt ein Verfahren zur heterogen katalysierten partiellen Gasphasenoxidation von Acrolein zu Acrylsäure, bei dem die Festbettkatalysatorschüttung aus wenigstens zwei räumlich aufeinanderfolgenden Festbettkatalysatorschüttungszonen besteht, wobei die volumenspezifische Aktivität beim Übergang in eine nachfolgende Schüttungszone sprunghaft zunimmt.

Die WO 2011/134932 offenbart einen Schalenkatalysator, bestehend aus einem hohlzylindrischen Trägerkörper sowie einer auf die äußere Oberfläche des Trägerkörpers aufgebrachten Schale aus katalytisch aktiver Oxidmasse, sowie ein Verfahren zur Herstellung von Acrylsäure durch gasphasenkatalytische Oxidation von Acrolein an einem Katalysatorfestbett, das den Schalenkatalysator umfasst. In den Ausführungsbeispielen werden nach 100 Betriebsstunden Selektivitäten der Acrylsäurebildung von bis zu 97,5 % erreicht.

Die Aufgabe bestand darin, einen Katalysator bereitzustellen, mit dem bei fortdauernd hohem Umsatz an Acrolein die Überoxidation zu $CO_x$ verringert und die Selektivitäten der Acrylsäurebildung erhöht werden können.

Diese Aufgabe wird gelöst durch einen Katalysator zur Herstellung einer $\alpha,\beta$-ungesättigten Carbonsäure durch Gasphasenoxidation eines $\alpha,\beta$-ungesättigten Aldehyds, umfassend einen Trägerformkörper mit einer darauf aufgebrachten Aktivmasse, dadurch gekennzeichnet, dass die Aktivmassebedeckung q

$$q = \frac{Q}{(100-Q)\,S_m}$$

höchstens 0,22 mg/mm$^2$ beträgt, wobei Q der Aktivmasseanteil des Katalysators in Gew.-% und $S_m$ die spezifische geometrische Oberfläche des Trägerformkörpers in mm$^2$/mg ist und die Aktivmasse eine die Elemente Mo und V enthaltende katalytisch aktive Multielementoxidmasse umfasst, wobei der molare Anteil des Elementes Mo an der Gesamtmenge aller von Sauerstoff verschiedenen Elemente der katalytisch aktiven Multielementoxidmasse 20 mol-% bis 80 mol-% beträgt, und das molare Verhältnis von in der katalytisch aktiven Multielementoxidmasse enthaltenem Mo zu in der katalytisch aktiven Multielementoxidmasse enthaltenem V, Mo/V, 15:1 bis 1:1 beträgt.

Die Aktivmassebedeckung q beträgt höchstens 0,22 mg/mm$^2$. Im Allgemeinen beträgt die Aktivmassebedeckung q wenigstens 0,10 mg/mm$^2$, bevorzugt wenigstens 0,15 mg/mm$^2$

Der Trägerformkörper weist vorzugsweise eine definierte geometrische Form auf.

Bevorzugte Trägerformkörper sind Ringe, Kugeln, Tabletten, gelochte Tabletten, Trilobe, gelochte Trilobe, Sternstränge, Sterntabletten, Wagenräder, Extrudate, Pillen, Zylinder und Hohlzylinder. Die Längstausdehnung (d.h. die längste geradlinige Direktverbindung zweier auf der Formkörperoberfläche befindlicher Punkte) des Trägerformkörpers beträgt vorteilhaft 1 bis 10 mm.

Besonders bevorzugte Trägerformkörper sind Hohlzylinder. Der hohlzylindrische Trägerformkörper weist vorzugsweise eine Höhe von 2 bis 5 mm und einen Außendurchmesser von 4 bis 8 mm auf, während der Halbwert der Differenz des Außendurchmessers und Innendurchmessers 1 bis 2 mm beträgt. Der Halbwert der Differenz des Außendurchmessers und Innendurchmessers entspricht der Wanddicke. Besonders bevorzugt ist eine Geometrie mit einem Außendurchmesser von 7 mm, einer Höhe von 3 mm und einem Innendurchmesser von 4 mm.

Der Trägerformkörper besteht vorzugsweise aus inertem Material. Inert bedeutet, dass sich das Material des Trägerformkörpers unter den Bedingungen der Gasphasenoxidation nicht wesentlich verändert und eine im Vergleich zur aufgebrachten Aktivmasse keine oder allenfalls vernachlässigbare katalytische Aktivität bezüglich der Gasphasenoxidation aufweist. Als inertes Material kommen insbesondere Aluminiumoxid, Siliciumdioxid, Siliciumcarbid, Zirkondioxid, Thoriumdioxid, Silicate wie Ton, Kaolin, Steatit, Bims, Aluminiumsilicat, und Magnesiumsilicat und deren Mischungen in Betracht. Steatit wird bevorzugt. Steatit des Typs C 220 wird besonders bevorzugt. Ganz besonders bevorzugt wird Steatit des Typs C 220 der Firma CeramTec.

Bevorzugt weist der Trägerformkörper eine deutlich ausgebildete Oberflächenrauhigkeit auf (z. B. Hohlzylinder mit Splittauflage). Mit Vorteil ist die Oberfläche des hohlzylindrischen Trägerformkörpers rau, da eine erhöhte Oberflächenrauhigkeit in der Regel eine erhöhte Haftfestigkeit der auf der Oberfläche der hohlzylindrischen Trägerformkörpers aufgebrachten Schale an Aktivmasse und/oder Vorläufermasse bedingt. Die Oberflächenrauhigkeit Rz beträgt vorzugsweise 30 bis 60 $\mu$m, besonders bevorzugt 40 bis 50 $\mu$m (bestimmt gemäß DIN 4768 Blatt 1 mit einem "Hommel Tester für DIN-ISO Oberflächenmessgrössen" der Fa. Hommelwerke).

Das inerte Material kann porös oder unporös sein. Vorzugsweise ist das inerte Material im Wesentlichen unporös (das Gesamtvolumen der Poren, bezogen auf das Volumen des Trägerkörpers, beträgt weniger als 1 Vol.-%). Die geometrische Dichte des inerten Materials liegt im Allgemeinen im Bereich von 0,5 bis 8,0 g/cm$^3$, bevorzugt 1,0 bis 7,0 g/cm$^3$, weiterhin bevorzugt 1,5 bis 6,0 g/cm$^3$, besonders bevorzugt 2,0 bis 5,0 g/cm$^3$ Die geometrische Dichte des chemisch inerten Materials wird berechnet, indem man die Masse des Trägerformkörpers durch dessen geometrisches Volumen teilt.

Das geometrische Volumen kann aus entsprechenden Messwerten der perfekten zugrundeliegenden geometrischen Formen berechnet werden. Z. B. kann das geometrische Volumen eines Hohlzylinders unter Zugrundelegung der Höhe H des Zylinders, des Außendurchmessers AD und des Durchmessers der Innenbohrung ID berechnet werden.

Der Aktivmasseanteil Q (in Gew.-%) des Katalysators ist die Masse der Aktivmasse, bezogen auf die Summe der Massen von Aktivmasse und Trägerformkörper. Zur Bestimmung der Masse der Aktivmasse kann man von der durch Wägung ermittelten Masse eines Katalysators (nach der Wärmebehandlung zur Entfernung des Bindemittels; siehe unten) die bekannte Masse des Trägerformkörpers subtrahieren. Zur Erhöhung der Messgenauigkeit kann man die Masse einer Vielzahl von Katalysatoren bzw. Trägerformkörpern bestimmen und mitteln. So kann die Masse der Aktivmasse einer definierten Anzahl an Katalysatoren durch Bestimmung der Gesamtmasse der Katalysatoren und Subtraktion des Trägerformkörpergewichts, das sich aus der Multiplikation des Trägerformkörpergewichts mit der Anzahl an Trägerformkörpern ergibt, bestimmt werden. Die Bestimmung des Aktivmassenanteils Q ist außerdem durch Abwaschen der Aktivmasse vom Trägerformkörper möglich. Dazu kann der beschichtete Katalysator beispielsweise mehrmals mit wässriger Ammoniaklösung ausgekocht und die entstehende Flüssigkeit abdekantiert werden. Der zurückbleibende Träger kann anschließend getrocknet werden. Der Aktivmassenanteil ergibt sich aus der Differenz von Katalysatormasse (vor Abwaschen der Aktivmasse bestimmt) und Trägermasse (bestimmt nach dem Abwaschen der Aktivmasse und Trocknung) bezogen auf die Katalysatormasse.

Der Trägermasseanteils des Katalysators in Gew.-% ist demnach (100-Q).

Die spezifische geometrische Oberfläche des Trägerformkörpers $S_m$ ist die auf die Masse des Trägerformkörpers bezogene geometrische Oberfläche des Trägerformkörpers.

Die geometrische Oberfläche kann aus entsprechenden Messwerten der perfekten zugrundeliegenden geometrischen Formen berechnet werden. Die geometrische Oberfläche ist eine idealisierte Größe und berücksichtigt nicht die durch die Porosität oder Oberflächenrauhigkeit der Formkörper bedingte Oberflächenvergrößerung.

Im Fall eines kugelförmigen Trägerformkörpers beträgt die geometrische Oberfläche

$4\pi r^2$

wobei r für den Radius des kugelförmigen Trägerformkörpers steht. Im Fall eines hohlzylindrischen Trägerformkörpers beträgt die geometrische Oberfläche

$$\frac{\pi}{2}\left((AD)^2-(ID)^2\right)+\pi(AD+ID)H$$

wobei H für die Höhe, AD für den Außendurchmesser und ID für den Innendruchmesser des hohlzylindrischen Trägerformkörpers steht.

Vorzugsweise beträgt die mittlere Dicke, der auf dem Trägerformkörper aufgebrachten Aktivmasse 50 bis 400 $\mu$m, bevorzugt 75 bis 350 $\mu$m, besonders bevorzugt 100 bis 300 $\mu$m und ganz besonders bevorzugt 100 bis 200 $\mu$m.

Vorzugsweise ist die Dicke der auf dem Trägerformkörper aufgebrachten Aktivmasse möglichst einheitlich. Die Dicke der aufgebrachten Aktivmassen ist zwischen verschiedenen Trägerformkörpern ebenfalls möglichst einheitlich.

Als Aktivmassen eignen sich katalytisch aktive Multielementoxidmassen, die die Elemente Mo und V enthalten, wobei der molare Anteil des Elementes Mo an der Gesamtmenge aller von Sauerstoff verschiedenen Elemente der katalytisch aktiven Multielementoxidmasse 20 mol-% bis 80 mol-% beträgt, das molare Verhältnis von in der katalytisch aktiven Multielementoxidmasse enthaltenem Mo zu in der katalytisch aktiven Multielementoxidmasse enthaltenem V, Mo/V, 15:1 bis 1:1 beträgt. Vorzugsweise enthält das Multielementoxid außerdem wenigstens eines der Elemente Nb und W; das entsprechende molare Verhältnis Mo/(Gesamtmenge aus W und Nb) beträgt vorzugsweise 80:1 bis 1:4.

Häufig enthalten solche Multielementoxidmassen noch Cu im entsprechenden molaren Verhältnis Mo/Cu von 30:1 bis 1:3.

Vorgenannte Multielementoxidmassen können neben den Elementen Mo, V, und gegebenenfalls Nb und/oder W oder Cu zusätzlich z.B. die Elemente Ta, Cr, Ce, Ni, Co, Fe, Mn, Zn, Sb, Bi, Alkali (Li, Na, K, Rb, Cs), H, Erdalkali (Mg, Ca, Sr, Ba), Si, Al, Ti und Zr enthalten. Natürlich kann die Multielementoxidaktivmasse aber auch nur aus den Elementen Mo, V, O, sowie Cu und gegebenenfalls W und/oder Nb bestehen. Sie eignen sich insbesondere als Aktivmassen für Katalysatoren zur heterogen katalysierten partiellen Gasphasenoxidation von Acrolein zu Acrylsäure.

Als Aktivmasse für Katalysatoren zur heterogen katalysierten partiellen Gasphasenoxidation von Acrolein zu Acrylsäure ganz besonders geeignete Massen umfassen ein Multielementoxidmassen der nachfolgenden allgemeinen Formel (I)

$$Mo_{12}V_aX^1_bX^2_cX^3_dX^4_eX^5_fO_n \qquad (I),$$

worin

$X^1$ für W, Nb, Ta, Cr und/oder Ce steht,
$X^2$ für Cu, Ni, Co, Fe, Mn und/oder Zn steht,
$X^3$ für Sb und/oder Bi steht,
$X^4$ für eines oder mehrere Alkali- und/oder Erdalkalimetalle und/oder N steht,
$X^5$ für Si, Al, Ti und/oder Zr steht,
a für eine Zahl im Bereich von 1 bis 6 steht,
b für eine Zahl im Bereich von 0,2 bis 4 steht,
c für eine Zahl im Bereich von 0 bis 18, vorzugsweise von 0,5 bis 18, steht
d für eine Zahl im Bereich von 0 bis 40 steht,
e für eine Zahl im Bereich von 0 bis 4 steht,
f für eine Zahl im Bereich von 0 bis 40 steht, und
n für den stöchiometrischen Koeffizienten des Elements Sauerstoff steht, der durch die stöchiometrischen Koeffizienten der von Sauerstoff verschiedenen Elemente sowie deren Ladungszahl in (I) bestimmt wird.

Vorzugsweise sind die Variablen innerhalb der vorgegebenen Bereiche mit der Maßgabe auszuwählen, dass der molare Anteil des Elementes Mo an der Gesamtmenge aller von Sauerstoff verschiedenen Elemente der Multielementoxidmasse (I) 20 mol-% bis 80 mol-% beträgt.

Die Multielementoxidmasse entspricht vorzugsweise der allgemeinen Formel (II)

$$Mo_{12}V_aW_bCu_cX^4_eX^5_fO_n \qquad (II)$$

worin

$X^4$ für eines oder mehrere Alkali- und/oder Erdalkalimetalle steht,
$X^5$ für eines oder merhrere Elemente aus der Gruppe Si, Al, Ti und Zr steht,
a für eine Zahl im Bereich von 2 bis 4, vorteilhaft für eine Zahl im Bereich von 2,5 bis 3,5 steht,
b für eine Zahl im Bereich von 0 bis 3, vorteilhaft für eine Zahl im Bereich von 0,2 bis 3, vorzugsweise für eine Zahl im Bereich von 0,5 bis 2, besonders bevorzugt für eine Zahl im Bereich von 0,75 bis 1,5 steht,
c für eine Zahl im Bereich von 0,5 bis 3, vorteilhaft für eine Zahl im Bereich von 0,7 bis 2,7, vorzugsweise für eine Zahl im Bereich von 0,9 bis 2,4, besonders bevorzugt für eine Zahl im Bereich von 1 bis 1,5 steht,
e für eine Zahl im Bereich von 0 bis 4, vorteilhaft für eine Zahl im Bereich von 0 bis 2, bevorzugt für eine Zahl im Bereich von 0 bis 1, besonders bevorzugt für eine Zahl im Bereich von 0 bis 0,2 steht,
f für eine Zahl im Bereich von 0 bis 40, vorteilhaft für eine Zahl im Bereich von 0 bis 15, bevorzugt für eine Zahl im Bereich von 0 bis 8, besonders bevorzugt für für 0 steht, und
n für den stöchiometrischen Koeffizienten des Elements Sauerstoff steht, der durch die stöchiometrischen Koeffizienten der von Sauerstoff verschiedenen Elemente sowie deren Ladungszahl in (II) bestimmt wird.

Elemente $X^4$ und $X^5$ sind nicht notwendigerweise Bestandteil der Aktivmasse der allgemeinen Formel (II). Sie wirken innerhalb der Aktivmasse im Allgemeinen wie inerte Verdünnungsmittel. Durch ihre Einarbeitung in die Aktivmasse kann die volumenspezifische Katalysatoraktivität auf ein gewünschtes Niveau eingestellt werden.

In einer Ausführungsform kann die Aktivmasse in Form eines feinteiligen Gemisches der die Elemente Mo und V enthaltenden Multielementoxidmasse, z.B. der Formel I oder II, mit einer Molybdänoxidquelle vorliegen, wie in der DE 10 2007 010 422 beschrieben. Die Molybdänoxidquelle ist geeigneterweise ausgewählt unter Oxiden des Molybdäns und Verbindungen des Molybdäns, aus denen sich unter Einwirkung von erhöhter Temperatur und molekularem Sauerstoff ein Oxid des Molybdäns bildet. Hierzu zählen Molybdänoxide wie $MoO_3$, $Mo_{18}O_{52}$, $Mo_8O_{23}$ und $Mo_4O_{11}$ oder Verbindungen wie Ammoniummolybdat $[(NH_4)_2MoO_4]$ sowie die Ammoniumpolymolybdate wie das Ammoniumhepta-molybdattetrahydrat $[(NH_4)_6Mo_7O_{24} \cdot 4H_2O]$ genannt. Ein alternatives Beispiel ist Molybdänoxidhydrat ($MoO_3 \cdot xH_2O$). $MoO_3$ ist eine bevorzugte Molybdänoxidquelle.

Die Körnung (Partikeldurchmesser, bzw. Partikeldurchmesserverteilung) der feinteiligen Molybdänoxidquelle ist erfindungsgemäß vorteilhaft mit jener des die Elemente Mo und V enthaltenden feinteiligen Multielementoxids identisch (dies ermöglicht eine besonders homogene Vermischung mit dem feinteiligen Multielementoxid). Dies gilt insbesondere dann, wenn die feinteilige Molybdänoxidquelle ein Molybdänoxid (insbesondere $MoO_3$) ist.

Die Mitverwendung der Molybdänoxidquelle kann der Deaktivierung des Katalysators im Verlauf einer heterogen katalysierten partiellen Gasphasenoxidation von Acrolein zu Acrylsäure präventiv entgegenwirken bzw. das Einsetzen der Deaktivierung hinauszögern.

Im Allgemeinen ist der Katalysator porös. Der Katalysator weist bevorzugt eine bestimmte Verteilung von Poren unterschiedlicher mittlerer Durchmesser auf. Der Volumenanteil $p_{vol}$ an Makroporen des Katalysators beträgt vorzugsweise zumindest 0,35, wobei $p_{vol}$ bestimmt ist durch

$$p_{vol} = \frac{V_{0,26-2}}{V_{0,02-6,5}}$$

worin

$V_{0,26-2}$ für das Volumen der Poren mit mittleren Durchmessern im Bereich von 0,26 bis 2 $\mu m$ steht, und

$V_{0,02-6,5}$ für das Volumen der Poren mit mittleren Durchmessern im Bereich von 0,02 bis 6,5 $\mu m$ steht.

Das Volumen der Poren mit mittleren Durchmessern im Nanometer- und Mikrometerbereich kann man durch Quecksilberporosimetrie (z.B. gemäß DIN Nr. 66133) bestimmen. Quecksilber verhält sich gegenüber den meisten Festkörpern als nicht benetzende Flüssigkeit. Daher wird Quecksilber nicht spontan von dem porösen Material absorbiert, sondern dringt nur unter einem äußeren Druck in die Poren der Festkörperprobe ein. Die Höhe dieses Drucks hängt von der Größe der Poren ab. Dieses Verhalten wird bei der Quecksilberporosimetrie ausgenutzt, um bei einem außen angelegten Druck über die volumetrisch erfasste Intrusion den Porendurchmesser zu erfassen.

Dabei wird das zuvor ausgegaste (um in der porösen Struktur gegebenenfalls enthaltene Flüssigkeit auszugasen) poröse System (die zu untersuchende Probe) in ein Quecksilberbad getaucht, dessen Druck verändert werden kann.

Da das Quecksilber das Probenmaterial nicht benetzt, muss das Quecksilber in die Poren der Probe gedrückt werden (beim jeweiligen Druck wird Gleichgewichtseinstellung abgewartet). Das Eindringen des Quecksilbers in Poren mit größerer Querschnittsfläche erfolgt bei vergleichsweise geringeren Drucken, während das Eindringen des Quecksilbers in engere Poren einen vergleichsweise höheren Druck erfordert. Unter der Annahme des Vorliegens von kreiszylindrischen Poren kann mit Hilfe der Washburn-Gleichung der äußere Druck, der erforderlich ist, um das flüssige Quecksilber gegen die Oberflächenspannung des Quecksilbers in die Poren eines entsprechenden Durchmessers zu drücken (zu intrudieren; Quecksilberintrusion), zum besagten Durchmesser in Beziehung gesetzt werden. Der im Rahmen der QuecksilberPorosimetrie-Untersuchung angewandte Druckbereich korreliert mit der (Band)Breite der erfassten Porendurchmesser.

Aus den bei 25 °C experimentell ermittelten Quecksilberintrusionskurven kann anschließend über die erfasste (Band)Breite der Porendurchmesser die Durchmesserverteilung der Poren, die innere Gesamtoberfläche der Poren und das innere Gesamtvolumen der Poren (das Gesamtintrusionsvolumen; das Porengesamtvolumen) rechnerisch extrahiert werden (vgl. Inauguraldissertation "Eigenschaften und Einsatzmöglichkeiten von Aerogelfenstern im Vergleich mit konventionellen sowie evakuierten Fenstern" von Georges Reber (1991), an der Philosophisch-naturwissenschaftlichen Fakultät der Universität Basel). Das unten beschriebene Messgerät Auto Pore IV 9520 der Firma Micromeritics umfasst diesbezüglich geeignete Standardrechenprogramme.

Ein erfindungsgemäßer Katalysator wird im Allgemeinen durch Aufbringen einer pulverförmigen Aktivmasse auf einen Trägerformkörper erhalten. Das erfindungsgemäße Verfahren zur Herstellung des Katalysators ist in Anspruch 7 definiert.

Die Herstellung der pulverförmigen Aktivmasse kann auf unterschiedliche Art und Weise erfolgen. In einer Ausführungsform erfolgt die Herstellung der Aktivmasse dadurch, dass man von Quellen der elementaren Konstituenten der Aktivmasse ein inniges Trockengemisch erzeugt, dieses bei Temperaturen von 350 bis 600 °C calciniert und es anschließend in Pulverform überführt.

Bevorzugte Quellen der elementaren Konstituenten der Aktivmasse sind Oxide von in der Aktivmasse enthaltenen Metallen. Als Quellen der elementaren Konstituenten der Aktivmasse kommen außerdem Verbindungen in Betracht, die durch Erhitzen, mindestens in Anwesenheit von Sauerstoff, in Oxide überführbar sind; insbesondere Halogenide, Nitrate, Formiate, Oxalate, Citrate, Acetate, Carbonate, Aminkomplexe, Ammoniumsalze und/oder Hydroxide von in der Aktivmasse enthaltenen Metallen.

Vorzugsweise erzeugt man das innige Trockengemisch durch inniges Vermischen der Quellen. Das innige Vermischen kann in trockener oder in nasser Form erfolgen. Erfolgt es in trockener Form, so werden die Quellen zweckmäßigerweise als feinteilige Pulver eingesetzt. Besonders innige Trockengemische werden beim Vermischen erhalten, wenn ausschließlich von in gelöster Form vorliegenden Quellen ausgegangen wird. Daher erfolgt das innige Vermischen der Quellen vorzugsweise in nasser Form. Vorzugsweise werden die Quellen in Form von Lösungen und/oder Suspensionen miteinander vermischt und die dabei resultierende nasse Mischung anschließend zum innigen Trockengemisch getrocknet. Als Lösungs- und/oder Suspendiermittel wird bevorzugt Wasser bzw. eine wässrige Lösung eingesetzt. Die Trocknung der nassen Mischung erfolgt vorzugsweise durch Sprühtrocknung mit Austrittstemperaturen von 100 bis 150 °C. Der trocknende Gasstrom ist vorzugsweise Luft oder molekularer Stickstoff.

Vor der Calcination kann das Trockengemisch (z.B. ein durch Sprühtrocknung erhaltenes Trockengemisch) einer Masseaufbereitung durch Mischen unterzogen werden. Unter dem Begriff Mischen versteht man Trockenmischen, Kneten und Rühren, gegebenenfalls unter Zugabe von Flüssigkeit. Durch das Mischen wird eine homogenisierte Masse mit engerer Teilchengrößenverteilung erhalten.

Besonders vorteilhaft wird die Mischoperation nach Zugabe einer Flüssigkeit, z.B. von Wasser, Essigsäure oder dergleichen, als Kneten durchgeführt, wobei man eine bildsame oder plastifizierte Masse erhält. Durch die dabei wirkenden Scherkräfte werden Agglomerate zerkleinert. Die bildsame Masse ist zur Strangextrusion geeignet und liefert stabile Stränglinge, die getrocknet werden können. Die getrockeneten Stränglinge eignen sich u.a. vorteilhaft zur Calcination im Drehrohr.

Die Calcination kann sowohl unter Inertgas als auch unter einer oxidativen Atmosphäre sowie auch unter reduzierender Atmosphäre durchgeführt werden. Vorzugsweise wird die Calcination unter einer oxidativen Atmosphäre durchgeführt. Als Inertgas kommen insbesondere Stickstoff, Wasserdampf, Edelgase, und deren Mischungen in Betracht. Die oxidative Atmosphäre enthält vorzugsweise Sauerstoff, insbesondere Luft. Die reduzierende Atmosphäre enthält vorzugsweise $H_2$, $NH_3$, CO, Methan, und/oder Acrolein. Die katalytische Aktivität der Aktivmasse zeigt in Abhängigkeit vom Sauerstoffgehalt der Calcinationsatmosphäre im Allgemeinen ein Optimum. Vorzugsweise beträgt der Sauerstoffgehalt der Calcinationsatmosphäre 0,5 bis 10 Vol.-%, besonders bevorzugt 1 bis 5 Vol.-%. Sauerstoffgehalte oberhalb und unterhalb der vorgenannten Grenzen verringern die resultierende katalytische Aktivität normalerweise. Die Calcinationsdauer kann einige Minuten bis einige Stunden betragen und nimmt üblicherweise mit der Höhe der Calcinationstemperatur ab. Ein gut geeignetes Calcinationsverfahren beschreibt z.B. die WO 95/11081.

Beim Calcinieren des Trockengemischs wird die Aktivmasse erhalten. Die Überführung in Pulverform erfolgt vorzugsweise durch Mahlen.

Die Aktivmasse ist vorzugsweise eine Aktivmasse der allgemeinen Formel (I) oder (II).

In einem erfindungsgemäßen Verfahren zur Herstellung des Katalysators beschichtet man den Trägerformkörper mit der Aktivmasse, indem man eine Vielzahl von Trägerformkörpern, eine pulverförmige Aktivmasse und ein flüssiges Bindemittel, ohne die pulverförmige Aktivmasse mit dem flüssigen Bindemittel zu sättigen, in einem Behälter durchmischt, wobei die Dauer des Beschichtungsvorgangs weniger als 30 Minuten beträgt. Man vermeidet die Sättigung der pulverförmigen Aktivmasse mit dem flüssigen Bindemittel, indem man das Verhältnis der Menge flüssigen Bindemittels zu der Menge pulverförmiger Aktivmasse so wählt, dass die Bindemittelmenge unterhalb des Flüssigkeitsaufnahmevermögens der pulverförmigen Aktivmasse bleibt.

Das Flüssigkeitsaufnahmevermögen von Pulvern kann z.B. bestimmt werden, indem man das Pulver in einem Rührwerk verwirbelt und Flüssigkeit auf das gerührte Pulver aufbringt und den zeitlichen Verlauf des Drehmomentes am Rührermotor misst. Aus der Flüssigkeitsmenge, die bis zum Maximum des Drehmoments auf das Pulver aufgetragen wurde, kann das Flüssigkeitsaufnahmevermögen des Pulvers berechnet werden.

Die pulverförmige Aktivmasse weist vorzugsweise einen Anteil von Partikeln mit einer Längstausdehnung oberhalb von 50 $\mu$m von weniger als 1 % auf.

Unter dem Begriff Bindemittel werden Substanzen verstanden, die die Haftung der Aktivmassepulverpartikel untereinander und/oder auf dem Trägermaterial dauerhaft oder temporär verbessern. Vorzugsweise verdampft oder sublimiert das Bindemittel bei der anschließenden Trocknung im Wesentlichen. Im erfindungsgemäßen Verfahren können als Bindemittel beispielsweise Polyole, wie Ethylenglycol, Propylenglycol, Butylenglycole, Glycerin oder Amide, wie Formamid, N,N-Dimethylformamid, N,N-Diethylformamid, N,N-Dibutylformamid, Acetamid, Pyrrolidon oder N-Methylpyrrolidon verwendet werden. Das flüssige Bindemittel ist vorzugsweise ausgewählt unter Wasser, Glycerin und Lösungen von Glycerin in Wasser. Bevorzugtes flüssiges Bindemittel ist eine Lösung von Glycerin in Wasser, die 20 bis 99 Gew.-% Wasser enthält. Besonders bevorzugtes flüssiges Bindemittel ist eine Lösung von Glycerin in Wasser, die 75 Gew.-% Wasser enthält.

Man legt die Trägerformkörper in dem Behälter vor und gibt die pulverförmige Aktivmasse und das flüssige Bindemittel getrennt voneinander über die Dauer der Beschichtung hinweg in den Behälter. So bringt man die pulverförmige Aktivmasse und das flüssige Bindemittel erst im Behälter miteinander in Kontakt. Man vereinigt die pulverförmige Aktivmasse und das flüssige Bindemittel vorzugsweise erst auf der Oberfläche der im Behälter vorgelegten Trägerformkörper. Dies gelingt, indem man das flüssige Bindemittel in den Behälter einsprüht und die pulverförmige Aktivmasse in einen außerhalb des Sprühkegels des flüssigen Bindemittels befindlichen Bereich des Behälters einträgt. So vermeidet man eine lokale Überladung der Pulverteilchen mit Flüssigkeit. Man kann die pulverförmige Aktivmasse und das flüssige Bindemittel beispielsweise durch kontinuierliche Zugabe oder durch zeitlich getrennte Zugabe von Teilmengen über die Dauer der Behandlung hinweg in den Behälter geben.

Das Durchmischen erfolgt vorzugsweise durch kontinuierliche Bewegung des Behälters. Die Bewegung ist vorzugsweise eine Rotationsbewegung.

Für eine Durchführung des vorstehend beschriebenen Verfahrens zur Herstellung des Katalysators eignet sich vor allem das in der DE-A 2909671 offenbarte Verfahrensprinzip (vgl. auch EP-A 714 700 sowie DE-A 10 2005 010 645) unter Verwendung des jeweils erwünschten flüssigen Bindemittels.

D. h., die zu beschichtenden Trägerformkörper, vorzugsweise hohlzylindrische Trägerformkörper, werden in einen vorzugsweise geneigten (der Neigungswinkel beträgt in der Regel 30 bis 90 °) rotierenden Drehbehälter (z. B. Drehteller oder Dragierkessel oder Dragiertrommel) gefüllt. Günstige Drehbehälter zu diesem Verwendungszweck sind insbesondere der Hi-Coater vom Typ HCF-100, der Fa. Freund Industrial Co., Ltd,Tokyo (JP) sowie der Hi-Coater vom Typ LH 100, der Fa. Gebrüder Lödige Maschinenbau GmbH, DE-Paderborn.

Der rotierende Drehbehälter führt die Trägerformkörper, vorzugsweise die hohlzylindrischen Trägerformkörper unter zwei in vorteilhaftem Abstand aufeinanderfolgend angeordneten Dosiervorrichtungen hindurch. Die erste der beiden Dosiervorrichtungen entspricht zweckmäßig einer Düse, durch die die im rotierenden Drehteller (Hi-Coater) rollenden Trägerformkörper mit dem flüssigen Bindemittel kontrolliert befeuchtet werden. Die zweite Dosiervorrichtung befindet sich anwendungstechnisch zweckmäßig außerhalb des Zerstäubungskegels des eingesprühten flüssigen Bindemittels und dient dazu, die pulverförmige Aktivmasse zuzuführen (z. B. über eine Schüttelrinne). Die Trägerformkörper nehmen die Aktivmasse auf, da sich die Aktivmasse durch die rollende Bewegung auf der Oberfläche der Trägerformkörper zu einer zusammenhängenden Schale verdichtet.

Bei Bedarf durchläuft der so grundbeschichtete vorzugswiese hohlzylindrische Trägerformkörper im Verlauf der darauffolgenden Umdrehung wiederum die Sprühdüse, wird dabei kontrolliert befeuchtet (gegebenenfalls mit einem anderen flüssigen Bindemittel), um im Verlauf der Weiterbewegung eine weitere Schicht (einer gegebenenfalls anderen) pulverförmigen Aktivmasse aufnehmen zu können usw. (eine Zwischentrocknung ist in der Regel nicht erforderlich). Die wenigstens teilweise Entfernung des verwendeten flüssigen Bindemittels kann z. B. gemäß der Lehre der EP-A 714 700 oder der Lehre der DE-A 10 2005 010 645 folgend durch abschließende Wärmezufuhr, z. B. durch Einwirkung von heißen Gasen wie $N_2$ oder Luft erfolgen (diese werden durch räumlich voneinander getrennt angebrachte netzartig gestaltete Wandelemente des Drehtellers, bzw. Dragierkessels, bzw. der Dragiertrommel (allgemein Drehbehälters) zu- und abgeführt).

Von Bedeutung für die beschriebene Ausführungsform des Beschichtungsverfahrens ist, dass die Befeuchtung der zu beschichtenden Oberfläche der Trägerformkörper in kontrollierter Weise vorgenommen wird. Kurz ausgedrückt heißt dies, dass man die Trägeroberfläche zweckmäßig so befeuchtet, dass diese zwar flüssiges Bindemittel adsorbiert aufweist, auf der Trägeroberfläche eine solche visuell aber nicht in Erscheinung tritt. Ist die Trägerformkörperoberfläche zu feucht, agglomeriert die feinteilige Aktiv- und/oder Vorläufermasse zu getrennten Agglomeraten, anstatt auf die Oberfläche aufzuziehen. Detailliertere Angaben hierzu finden sich in der DE-A 2909671, in der EP-A 714 700 sowie in der DE-A 10 2005 010 645. Ein Vorzug der beschriebenen Verfahrensweise besteht darin, dass die Entfernung des verwendeten flüssigen Bindemittels in vergleichsweise kontrollierter Weise z. B. durch Verdampfen und/oder Sublimieren vorgenommen werden kann. Im einfachsten Fall kann dies wie bereits ausgeführt durch die Einwirkung heißer Gase entsprechender Temperatur (häufig 50 bis 150 °C) erfolgen. Eine solche Einwirkung heißer Gase bewirkt im Allgemeinen eine Vortrocknung.

Die Entfernung des Bindemittels kann in einer Trockenvorrichtung beliebiger Art (z. B. in einem Bandtrockner) und/oder erst im Katalysatorfestbett des Rohrbündelreaktors befindlich erfolgen, wie es z. B. die DE-A 10 2005 010 645 empfiehlt. Vorzugsweise erhält man den erfindungsgemäßen Katalysator, indem man das flüssige Bindemittel vom beschichteten Trägerformkörper durch Trocknung bei einer Temperatur im Bereich von 150 bis 400 °C, bevorzugt 250 bis 350 °C entfernt. Die Trocknung führt man vorzugsweise in einem Luftstrom durch. Vorzugsweise beträgt die Dauer der Trocknung 0,5 bis 8 h, bevorzugt 1 bis 4 h.

Gegenstand der Erfindung ist auch ein Verfahren zur Herstellung einer $\alpha,\beta$-ungesättigten Carbonsäure durch Gasphasenoxidation eines $\alpha,\beta$-ungesättigten Aldehyds mit molekularem Sauerstoff an einem Katalysatorfestbett, das eine Schüttung eines erfindungsgemäßen Katalysator umfasst. Vorzugsweise bringt man den molekularen Sauerstoff und den $\alpha,\beta$-ungesättigten Aldehyd mit dem Katalysatorfestbett in Kontakt, indem man den molekularen Sauerstoff und den $\alpha,\beta$-ungesättigten Aldehyd über das Katalysatorfestbett führt. Vorzugsweise führt man ein Reaktionsgas, das den molekularen Sauerstoff und den $\alpha,\beta$-ungesättigten Aldehyd enthält, über das Katalysatorfestbett und setzt das Reaktionsgas so zu einem Produktgas um.

Der $\alpha,\beta$-ungesättigte Aldehyd ist vorzugsweise ausgewählt unter 3 bis 6 (d. h., 3, 4, 5 oder 6) C-Atome enthaltenden $\alpha,\beta$-ungesättigten Aldehyden, insbesondere unter Acrolein und Methacrolein. Besonders bevorzugt ist der $\alpha,\beta$-ungesättigte Aldehyd Acrolein. Das Verfahren eignet sich besonders zur Herstellung von $\alpha,\beta$-ungesättigten Carbonsäuren, insbesondere zur Oxidation von Acrolein zu Acrylsäure und von Methacrolein zu Methacrylsäure. Vorzugsweise handelt es sich um ein Verfahren zur Herstellung von Acrylsäure durch Gasphasenoxidation von Acrolein.

Der molekulare Sauerstoff wird dem Verfahren vorzugsweise in Form von Luft zugeführt.

Der Anteil des im Reaktionsgas enthaltenen $\alpha,\beta$-ungesättigten Aldehyds wird im Allgemeinen 3 bis 15 Vol.-%, vorzugsweise 4 bis 10 Vol.-%, bevorzugt 5 bis 8 Vol.-% betragen, jeweils bezogen auf das Reaktionsgas.

Vorzugsweise enthält das Reaktionsgas außerdem mindestens ein von Wasserdampf verschiedenes inertes Verdünnungsgas. Darunter werden solche Gase verstanden, die im Verlauf der Gasphasenoxidation zu mindestens 95 mol-%, vorzugsweise zu mindestens 98 mol-% chemisch unverändert erhalten bleiben. Beispiele für inerte Verdünnungsgase sind $N_2$, $CO_2$ und Edelgase wie Ar. Als inertes Verdünnungsgas wird vorzugsweise molekularer Stickstoff eingesetzt. Das inerte Verdünnungsgas kann mindestens 20 Vol.-%, bevorzugt mindestens 40 Vol.-%, weiterhin bevorzugt mindestens 60 Vol.-%, besonders bevorzugt mindestens 80 Vol.-%, ganz besonders bevorzugt mindestens 95 Vol.-% molekularen Stickstoff enthalten.

Das Reaktionsgas kann außerdem Wasserdampf enthalten.

Das Reaktionsgas kann außerdem Kreisgas enthalten. Unter Kreisgas wird das Restgas verstanden, das verbleibt, wenn man aus dem Produktgas der Gasphasenoxidation $\alpha,\beta$-ungesättigte Carbonsäure im Wesentlichen selektiv abtrennt.

Vorzugsweise bildet das erfindungsgemäße Verfahren zur Herstellung der $\alpha,\beta$-ungesättigten Carbonsäure die zweite Stufe einer zweistufigen Gasphasenoxidation eines Alkens zu der $\alpha,\beta$-ungesättigten Carbonsäure. Im Rahmen einer solchen zweistufigen Gasphasenoxidation wird das Produktgas der ersten Stufe vorzugsweise der zweiten Stufe zugeführt. Vor der Zuführung in die zweite Stufe kann man das Produktgas der ersten Stufe beispielsweise abkühlen und/oder Sauerstoff zugeben (Sekundärsauerstoffzugabe, bevorzugt ist die Zugabe von Luft). Das Kreisgas wird vorzugsweise in die erste der beiden Stufen geführt.

Im Reaktionsgas beträgt das molare Verhältnis von $O_2$:$\alpha,\beta$-ungesättigtem Aldehyd vorzugsweise 1 bis 3, bevorzugt 1 bis 2, besonders bevorzugt 1 bis 1,5.

Das Reaktionsgas enthält vorzugsweise $\alpha,\beta$-ungesättigten Aldehyd : Sauerstoff: Wasserdampf : von Wasserdampf verschiedenem inertem Verdünnungsgas in einem Volumenverhältnis von 1:(1 bis 3):(0 bis 20):(3 bis 30), vorzugsweise von 1:(1 bis 3):(0,5 bis 10):(7 bis 10).

Vorzugsweise beträgt die Belastung der Schüttung mit $\alpha,\beta$-ungesättigtem Aldehyd höchstens 600 Nl/(lh), bevorzugt höchstens 300 Nl/(lh), besonders bevorzugt höchstens 250 Nl/(lh), am meisten bevorzugt höchstens 200 Nl/(lh). Vorzugsweise beträgt die Belastung der Schüttung mit $\alpha,\beta$-ungesättigtem Aldehyd wenigstens 30 Nl/(lh), bevorzugt wenigstens 70 Nl/(lh), besonders bevorzugt wenigstens 90 Nl/(lh), am meisten bevorzugt wenigstens 120 Nl/(lh). Unter der in Nl/(lh) ausgedrückten Belastung der Schüttung mit $\alpha,\beta$-ungesättigtem Aldehyd wird die Menge an $\alpha,\beta$-ungesättigtem Aldehyd in Normlitern verstanden, die als Bestandteil des Reaktionsgases pro Stunde pro Liter Schüttung über das Katalysatorfestbett geführt wird. Ein Normliter (Nl) ist das Volumen in Litern, das die molare Menge eines idealen Gases, die der molaren Menge an $\alpha,\beta$-ungesättigtem Aldehyd entspricht, bei Normalbedingungen, d. h., bei 25 °C und 1 bar, einnehmen würde.

Im Allgemeinen herrscht im Reaktionsgas ein Gesamtdruck von 0,5 bis 100 bar, bevorzugt von 1 bis 5 bar, insbesondere von 1 bis 3 bar. Alle Druck-Angaben in dieser Schrift beziehen sich auf absolute Drücke.

Vorzugsweise führt man das Verfahren zur Herstellung der $\alpha,\beta$-ungesättigten Carbonsäure in einem Rohrbündelreaktor durch, dessen Reaktionsrohre mit dem Katalysatorfestbett befüllt sind.

Die Schüttung kann beispielsweise ausschließlich aus erfindungsgemäßen Katalysatoren bestehen. In der Schüttung können auch weitgehend homogene Gemische aus erfindungsgemäßen Katalysatoren und Verdünnungsformkörpern, die sich bezüglich der Gasphasenoxidation im Wesentlichen inert verhalten, vorliegen. Als Materialien für die Verdünnungsformkörper kommen z. B. poröse oder unporöse Aluminiumoxide, Siliciumdioxid, Zirkondioxid, Siliciumcarbid, Silikate wie Magnesium- oder Aluminiumsilikat und/oder Steatit (z. B. vom Typ C220 der Fa. CeramTec, DE) in Betracht.

Die Geometrie der Verdünnungsformkörper kann im Prinzip beliebig sein. D. h., es können beispielsweise Ringe, Kugeln, Tabletten, gelochte Tabletten, Trilobe, gelochte Trilobe, Sternstränge, Sterntabletten, Wagenräder, Extrudate, Pillen, Zylinder und Hohlyzylinder sein.

Bei dem Rohrbündelreaktor handelt es sich bevorzugt um einen Zweizonenrohrbündelreaktor. Einen bevorzugten Zweizonenrohrbündelreaktors offenbart die DE-C 28 30 765. Aber auch die in der DE-C 25 13 405, der US-A 3147084, der DE-A 22 01 528, der EP-A 383224 und der DE-A 29 03 582 offenbarten Zweizonenrohrbündelreaktoren sind geeignet.

Um die Reaktionsrohre werden im Zweizohnenröhrbündelreaktor vorzugsweise zwei voneinander im Wesentlichen räumlich getrennte Temperiermedien geführt. Bei den Temperiermedien handelt es sich vorzugsweise um Salzschmelzen. Die Eingangstemperatur des Temperiermediums wird vorzugsweise auf 230 bis 300 °C, bevorzugt auf 240 bis 290 °C, besonders bevorzugt auf 250 bis 285 °C eingestellt. Man kann das Temperiermedium über die jeweilige Temperierzone im Gleichstrom oder im Gegenstrom zum Reaktionsgasgemisch führen. Innerhalb der Temperierzone wird das Temperiermedium bevorzugt mäanderförmig geführt. Die Fließgeschwindigkeit der Temperiermedien wird innerhalb der jeweiligen Temperierzone vorzugsweise so gewählt, dass die Temperatur des Wärmeaustauschmittels von der Eintrittsstelle in die Temperaturzone bis zur Austrittstelle aus der Temperaturzone um 0 bis 15 °C, häufig 1 bis 10 °C, oder 2 bis 8 °C, oder 3 bis 6 °C ansteigt.

In einer bevorzugten Ausführungsform umfasst das Katalysatorfestbett zumindest zwei aufeinanderfolgende Reaktionszonen, wobei die Schüttung zumindest in der zum Reaktoreingang nächsten Reaktionszone einen erfindungsgemäßen Katalysator umfasst.

In einer anderen bevorzugten Ausführungsform umfasst das Katalysatorfestbett zumindest zwei aufeinanderfolgende Reaktionszonen, wobei die Schüttung zumindest in der Reaktionszone, in der die höchste lokale Temperatur auftritt, einen erfindungsgemäßen Katalysator umfasst.

Die einzelnen Reaktionszonen unterscheiden sich voneinander in wenigstens einer Eigenschaft, ausgewählt unter dem Gehalt an inerten Verdünnungsformkörpern, Gestalt der Katalysatoren, Raumerfüllungsgrad der Katalysatoren, Aktivmasseanteil der Katalysatoren und chemischer Zusammensetzung der Aktivmasse.

In Folge der unterschiedlichen Eigenschaften kann sich die volumenspezifische Katalysatoraktivität einer Reaktionszone von der volumenspezifischen Katalysatoraktivität einer anderen Reaktionszone unterscheiden. Vorzugsweise nimmt die volumenspezifische Katalysatoraktivität vom Reaktoreingang zum Reaktorausgang von einer Reaktionszone zur nächsten zu.

Die (relative) volumenspezifische Katalysatoraktivität kann als Reaktionsgeschwindigkeit, bezogen auf das Katalysatorschüttungsvolumen, unter im Übrigen konstanten Bedingungen ermittelt werden.

Die volumenspezifische Katalysatoraktivität kann durch Verdünnung des Katalysators mit Verdünnungsformkörpern variiert werden. Alternativ oder zusätzlich kann die volumenspezifische Katalysatoraktivität durch Variation des Aktivmasseanteils eingestellt werden.

Vorzugsweise ist die Aktivmassenraumdichte, d.h. die Menge Aktivmasse in g pro Leerraumvolumen der Reaktionszone in Liter, in der dem Reaktoreingang nächsten Reaktionszone geringer, als in der dem Reaktorausgang nächsten Reaktionszone.

Ab einem bestimmten Betriebszeitpunkt geht mit zunehmender Betriebsdauer eine zunehmende Minderung der Qualität der Katalysatorbeschickung einher. In einer bevorzugten Ausführungsform entnimmt man zur Rückgewinnung der Qualität der Schüttung nicht die gesamte gebrauchte Schüttung, sondern nur die Teilmenge der Schüttung, in der die höchste lokale Temperatur auftritt, und ersetzt diese durch eine frische Schüttung. Z. B. ersetzt man die Schüttung derjenigen Reaktionszone, in der die höchste lokale Temperatur auftritt, durch eine frische Schüttung und behält die Schüttung in den in Strömungsrichtung des Reaktionsgases stromabwärts gelegenen Reaktionszonen bei.

In der Regel verfügt der Rohrbündelreaktor zusätzlich über Thermorohre zur Bestimmung der Gastemperatur im Katalysatorbett. In zweckmäßiger Weise wird der Innendurchmesser der Thermorohre und der Durchmesser der innenliegenden Aufnahmehülse für das Thermoelement so gewählt, dass das Verhältnis von Reaktionswärme entwickelndem Volumen zu Wärme abführender Oberfläche bei Thermorohren und Reaktionsrohren gleich oder nur geringfügig unterschiedlich ist.

Der Druckverlust sollte bei Reaktionsrohren und Thermorohren, bezogen auf gleiche GHSV, gleich sein. Ein Druckverlustausgleich beim Thermorohr kann z. B. durch Zusatz von versplittetem Katalysator zu den Katalysatoren erfolgen. Dieser Ausgleich erfolgt zweckmäßig über die gesamte Thermorohrlänge homogen. Im Übrigen kann die Befüllung von Thermorohren wie in der EP-A 873783 beschrieben gestaltet werden.

Mit den in den Thermorohren gemessenen Temperaturen lassen sich die höchste lokale Temperatur des Katalysatorfestbetts und deren Lage im Katalysatorfestbett bestimmen.

Figuren

Figur 1a zeigt die kumulative Partikelgrößenverteilung des feinteiligen Pulvers P. Dabei zeigt die Abszisse in logarithmischem Maßstab den Partikeldurchmesser in $\mu$m. Der zu einem bestimmten Partikeldurchmesser gehörige Ordinatenwert auf der Verteilungskurve zeigt den prozentualen Anteil des Gesamtpartikelvolumens, der aus Partikeln des jeweiligen Partikelduchmessers oder eines kleineren Partikeldurchmessers besteht.

Figur 1b zeigt die differentielle Partikelgrößenverteilung des feinteiligen Pulvers P. Dabei zeigt die Abszisse in logarithmischem Maßstab den Partikeldurchmesser in $\mu$m. Der zu einem bestimmten Partikeldurchmesser gehörige Ordinatenwert auf der Verteilungskurve zeigt den prozentualen Anteil des Gesamtpartikelvolumens, der aus Partikeln des jeweiligen Partikelduchmessers besteht.

Figur 2 zeigt ein Diffraktogramm des feinteiligen Pulvers P

Figur 3 zeigt die Porendurchmesserverteilung der Poren der Aktivmassenschale von C1.

Figur 4 zeigt die Porendurchmesserverteilung der Poren der Aktivmassenschale von C2.

Figur 5 zeigt die Porendurchmesserverteilung der Poren der Aktivmassenschale von C3.

Figur 6 zeigt die Porendurchmesserverteilung der Poren der Aktivmassenschale von C4.

Figur 7 zeigt die Porendurchmesserverteilung der Poren der Aktivmassenschale von C5.

Figur 8 zeigt die Porendurchmesserverteilung der Poren der Aktivmassenschale von C6.

Figur 9 zeigt die Porendurchmesserverteilung der Poren der Aktivmassenschale von C7.

Figur 10 zeigt die Porendurchmesserverteilung der Poren der Aktivmassenschale von C8.

Figur 11 zeigt die Porendurchmesserverteilung der Poren der Aktivmassenschale von C9.

Figur 12 zeigt die Porendurchmesserverteilung der Poren der Aktivmassenschale von C10.

Figur 13 zeigt die Porendurchmesserverteilung der Poren der Aktivmassenschale von C11.

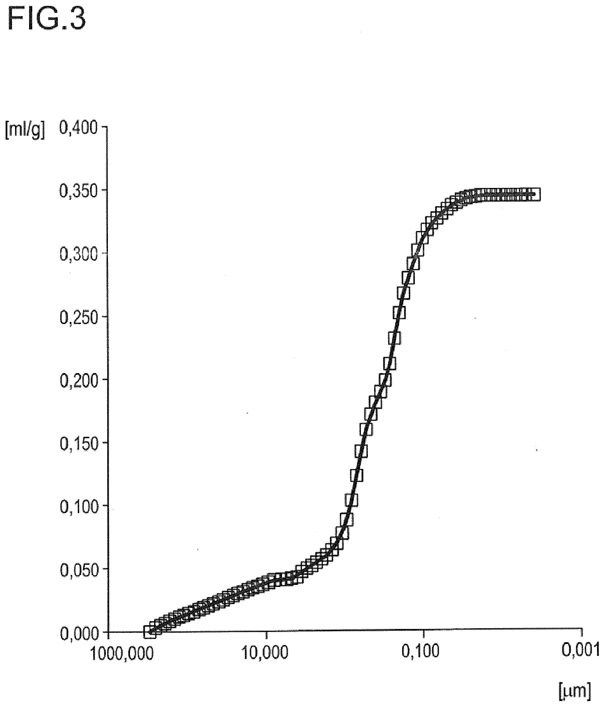

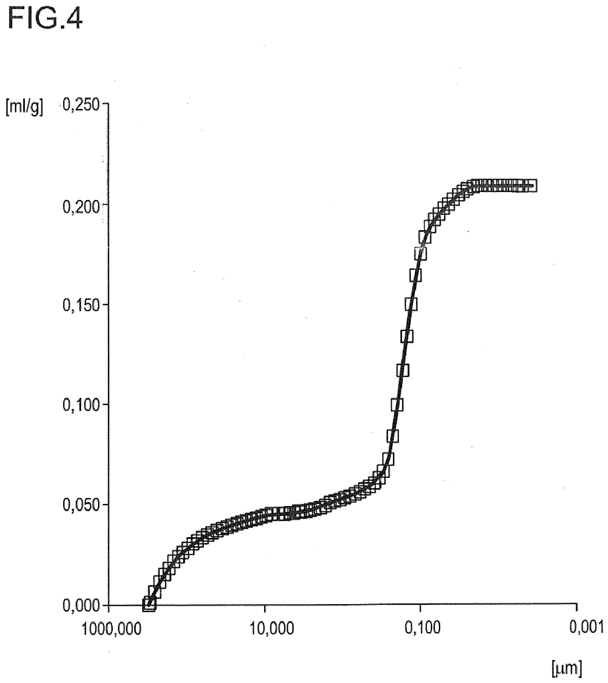

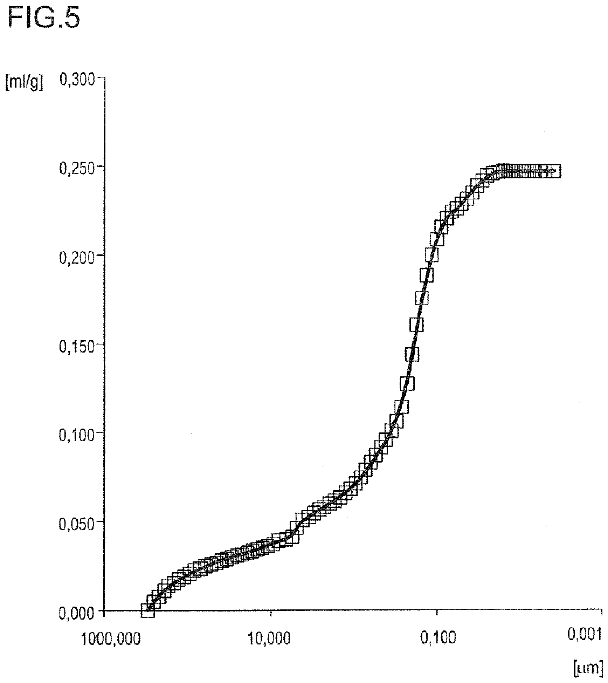

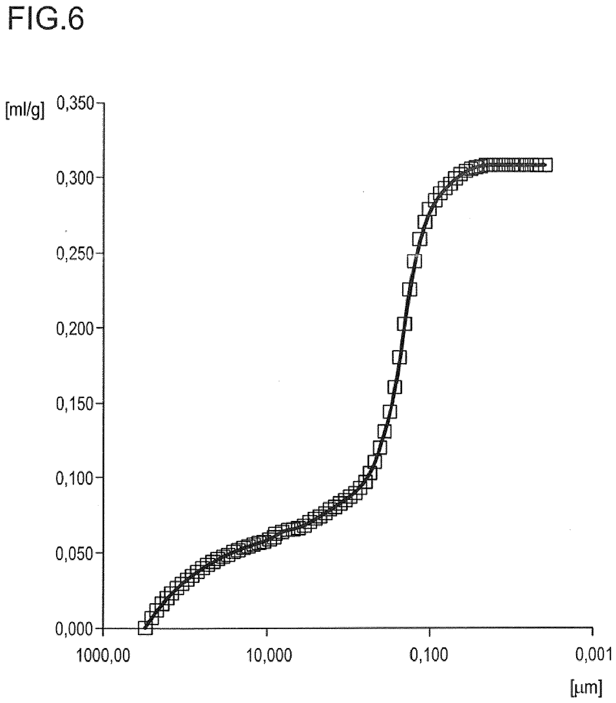

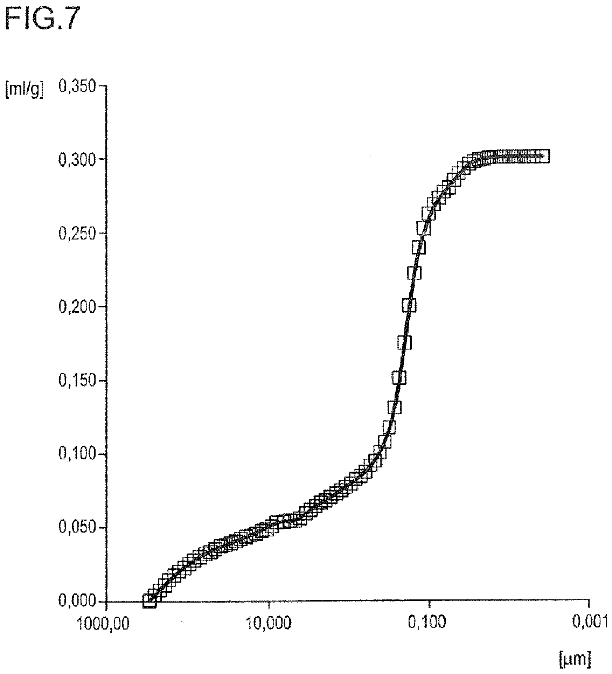

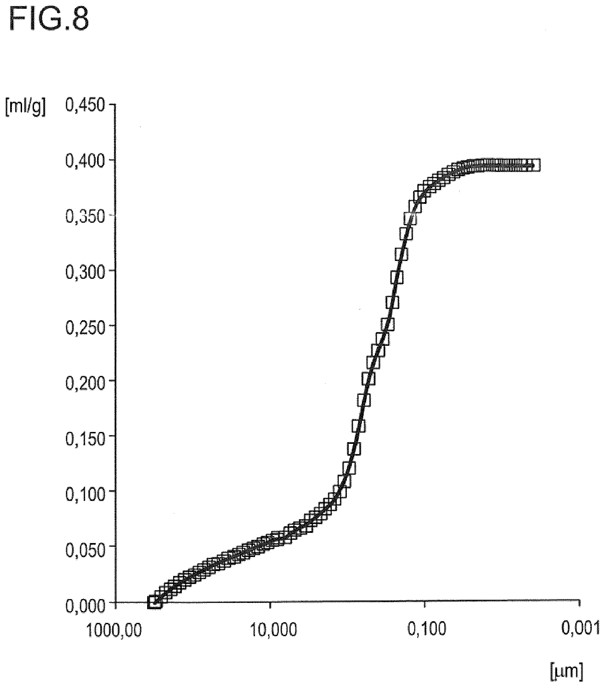

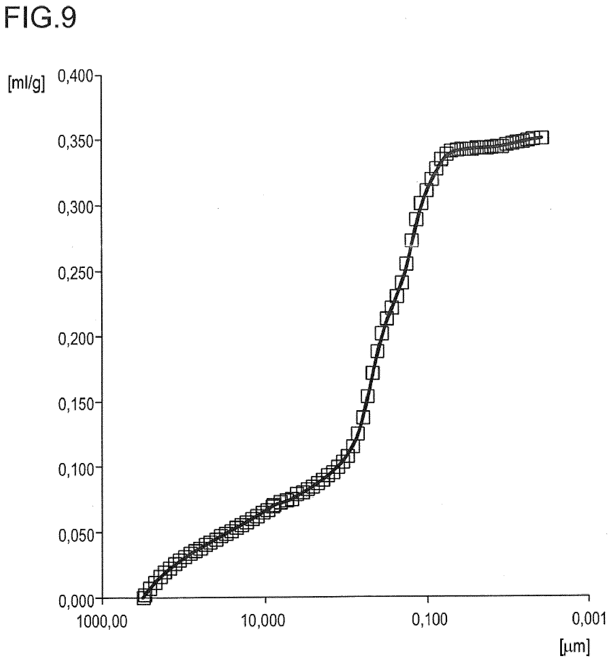

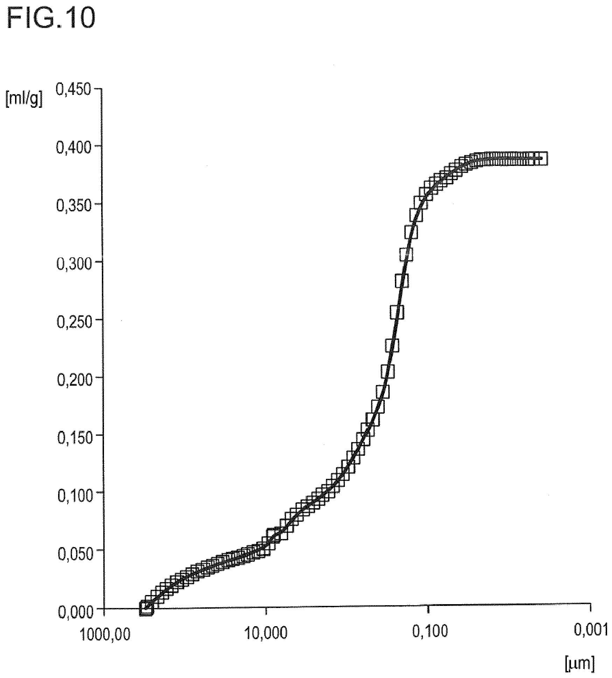

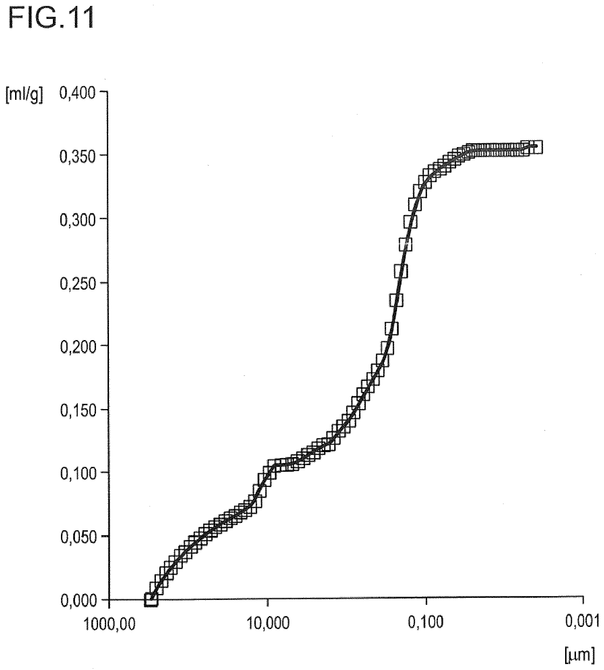

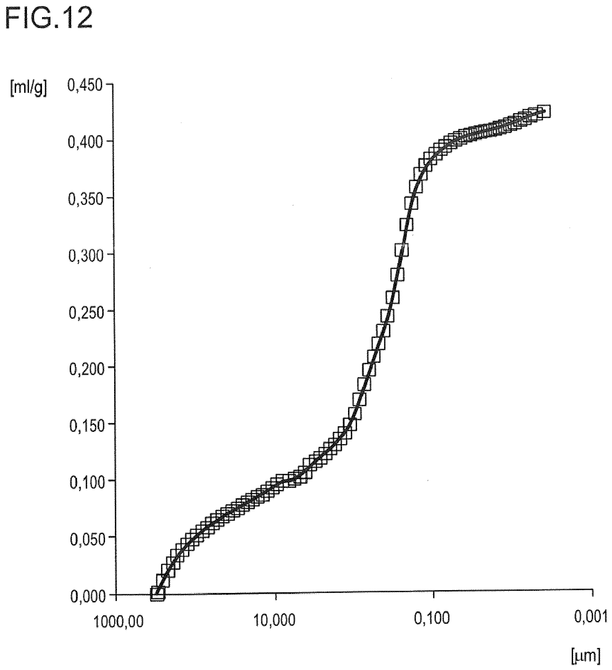

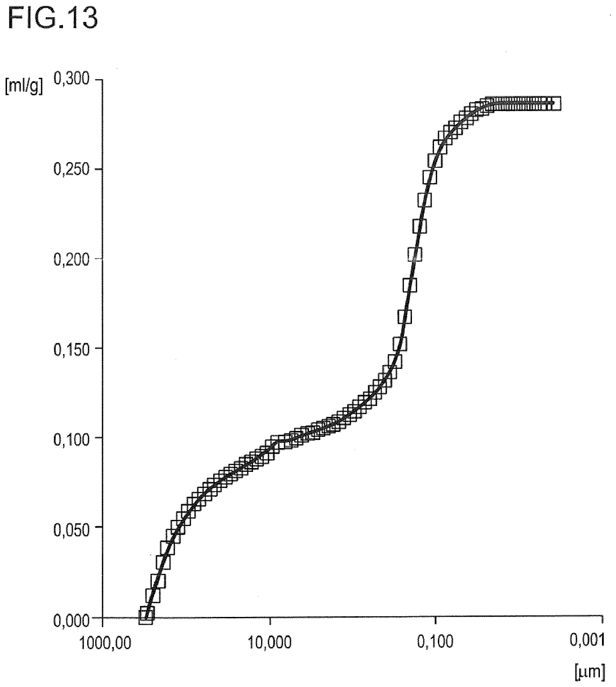

In Figuren 3 bis 13 ist auf der Abszisse der jeweilige Porendurchmesser in $\mu$m aufgetragen (logarithmische Auftragung zur Basis 10). Auf der Ordinate ist in (ml/g Aktivmasse) das Integral über die individuellen Beiträge der einzelnen Porendurchmesser zum spezifischen Porengesamtvolumen (der kumulative Beitrag zum spezifischen Porengesamtvolumen) aufgetragen (Kurve □). Der Endpunkt ist das auf die Aktivmasse bezogene (spezifische) Porengesamtvolumen (Gesamtintrusionsvolumen).

**Beispiele**

**Herstellung von Katalysatoren**

**A) Herstellung einer Vorläufermasse**

In einem wassertemperierten 1,75-m$^3$-Doppelmantelbehälter aus Edelstahl mit einem Balkenrührer wurden 8,2 kg Kupferacetathydrat (Gehalt: 32,0 Gew.-% Cu, Zugaberate 50 kg/h, Fa. Goldschmidt) in 274 l Wasser bei Raumtemperatur (~25°C) unter Rühren (Drehzahl: 70 Umdrehungen/min) gelöst. Es wurde eine Lösung 1 erhalten. Diese wurde weitere 30 min gerührt.

Räumlich getrennt davon wurden in einem wassertemperierten 1,75-m$^3$-Doppelmantelbehälter aus Edelstahl mit einem Balkenrührer (Drehzahl: 70 Umdrehungen/min) 614 l Wasser vorgelegt, auf 40°C erwärmt und 73 kg Ammoniumheptamolybdattetrahydrat (81,5 Gew.-% $MoO_3$, Zugaberate 300 kg/h, Fa. H.C. Starck GmbH) unter Beibehalt der 40°C eingerührt. Dann wurde der Behälterinhalt unter Rühren innerhalb von 30 min auf 90°C erwärmt und unter Beibehalt dieser Temperatur nacheinander und in der genannten Reihenfolge 12,1 kg Ammoniummetavanadat (77,6 % $V_2O_5$, Zugaberate 150 kg/h, Nachrührzeit nach Zugabe 40 min) sowie 10,7 kg Ammoniumparawolframattheptahydrat (89,6 Gew.-% $WO_3$, Zugaberate 50 kg/h, Nachrührzeit nach Zugabe 30 min) eingerührt. Es wurde eine Lösung 2 erhalten.

Die Lösung 2 wurde auf 80°C abgekühlt und anschließend die Lösung 1 zügig bei einer Rührerdrehzahl des Balkenrührers von 70 Umdrehungen/min in die Lösung 2 überführt und eingerührt. Das erhaltene Gemisch wurde mit 133 l einer 25 gew.%-igen wässrigen $NH_3$-Lösung versetzt, die eine Temperatur von 25°C aufwies. Unter Rühren entstand eine klare Lösung, die kurzzeitig eine Temperatur von 65°C und einen pH-Wert von 8,5 aufwies. Sie wurde in einen weiteren wassertemperierten 1,75-m$^3$-Doppelmantelbehälter aus Edelstahl mit einem Balkenrührer abgelassen. Der Behälterinhalt wurde auf 80°C aufgeheizt, bei einer Rührerdrehzahl von 40 Umdrehungen/min gerührt und im Kreislauf betrieben. Der ph-Wert des Behälterinhalts wurde mittels automatischer Zugabe einer 25gew.%-igen wässrigen $NH_3$-Lösung auf einem Wert von 8,5 gehalten. Der Behälterinhalt wurde in den Drehscheibensprühturm vom Typ FS 15 der Firma Niro (Dänemark) gepumpt und im Heißluftgleichstrom bei einer Gaseintrittstemperatur von 350 $\pm$ 10 °C,

einer Scheibendrehzahl von 15000 U/min und einem Verbrennungsluftvolumenstrom von 2300 Nm$^3$/h getrocknet, wobei im Sprühturm ein Unterdruck von 1 mbar aufrechterhalten wurde. Der dem Sprühturm zudosierte Flüssigkeitsvolumenstrom wurde dabei so geregelt, dass eine einer Gasaustrittstemperatur von 110 $\pm$ 5°C erreicht wurde. Das resultierende Sprühpulver wies einen Partikeldurchmesser von 2 bis 50 $\mu$m auf und einen Glühverlust von 21 $\pm$ 2 Gew.-%. Der Glühverlust wurde bestimmt durch Erhitzen im Porzellantiegel(3 h bei 600 °C) unter Luft. Der Porzellantiegel wurde zuvor bei 900 °C bis zur Gewichtskonstanz geglüht. Die Abfüllung des Sprühpulvers erfolgte in Spezialcontainer oder Spezialfässer (200 Liter) mit Kunststoffinlet. Zum Abtrennen von Brocken wurde dabei ein Siebeinsatz verwendet.

75 kg von so erhaltenem Sprühpulver wurden in einem Kneter der Fa. AMK (Aachener Misch- und Knetmaschinen Fabrik) vom Typ VM 160 (Sigma Schaufeln) bei einer Schneckendrehzahl von 15 Umdrehungen/min dosiert. Anschließend wurden 6,5 l Essigsäure (100gew.-%ig, Eisessig) und 5,2 l Wasser in den Kneter bei einer Schneckendrehzahl von 15 Umdrehungen/min dosiert. Nach einer Knetdauer von 4 bis 5 Minuten (Drehzahl der Schnecke: 20 Umdrehungen/min) wurden weitere 6,5 l Wasser zugegeben und der Knetprozess bis zum Ablauf von 30 Minuten fortgesetzt (Knettemperatur ca. 40 bis 50°C). Bei der Knetung wurde die Stromaufnahme beobachtet. Bei Überschreiten einer Stromaufnahme von 25% wurde bei Bedarf noch etwa 1 l Wasser dem Knetgut zugegeben. Danach wurde das Knetgut in einen Extruder entleert und mittels des Extruders (Fa. Bonnot Company (Ohio), Typ: G 103-10/D7A-572K (6" Extruder W Packer) zu Strängen (Länge: 1-10 cm; Durchmesser 6 mm) geformt. Auf einem 3-zonigen Bandtrockner wurden die Stränge bei einer Bandgeschwindigkeit von 10 cm/min und einer resultierenden Verweilzeit von 64 min sowie einer Gaseintrittstemperatur von 155°C getrocknet. Die Erwartungswerte der Gastemperaturen betragen in der Zone 1 90-95°C, in Zone 2 ca. 115°C und in Zone 3 ca. 125°C.

**B) Herstellung einer Aktivmasse der Formel Mo$_{12}$V$_3$W$_{1,2}$CU$_{1,2}$O$_n$**

Die Durchführung der thermischen Behandlung erfolgte im in der DE 10360057A1 beschriebenen Drehrohrofen unter den folgenden Bedingungen:

die thermische Behandlung erfolgte diskontinuierlich mit einer Materialgutmenge von 306 kg, die wie unter A) beschrieben hergestellt worden war;
der Neigungswinkel des Drehrohres zur Horizontalen betrug $\approx$ O°;
das Drehrohr rotierte mit 1,5 Umdrehungen/min rechts herum;
während der gesamten thermischen Behandlung wurde durch das Drehrohr ein Gasstrom von 205 Nm$^3$/h geführt, der (nach Verdrängung der ursprünglich enthaltenen Luft) wie folgt zusammengesetzt war und an seinem Ausgang aus dem Drehrohr durch weitere 25 Nm$^3$/h Sperrgasstickstoff ergänzt wurde: 80 Nm$^3$/h zusammengesetzt aus Grundlast-Stickstoff und im Drehrohr freigesetzten Gasen, 25 Nm$^3$/h Sperrgasstickstoff, 30 Nm3/h Luft und 70 Nm$^3$/h rezirkuliertes Kreisgas. Der Sperrgas-Stickstoff wurde mit einer Temperatur von 25°C zugeführt. Das Gemisch der anderen Gasströme wurde aus dem Erhitzer kommend jeweils mit der Temperatur ins Drehrohr geführt, die das Materialgut im Drehrohr jeweils aufwies.

innerhalb von 10 h wurde die Materialguttemperatur von 25°C im Wesentlichen linear auf 300°C erhöht; anschließend wurde die Materialguttemperatur innerhalb von 2 h im Wesentlichen linear auf 360°C erhöht; nachfolgend wurde die Materialguttemperatur innerhalb von 7 h im Wesentlichen linear auf 350°C gesenkt; dann wurde die Materialguttemperatur innerhalb von 2 h im Wesentlichen linear auf 420°C erhöht und diese Materialguttemperatur während 30 min gehalten; dann wurden im durch das Drehrohr geführten Gasstrom die 30 Nm$^3$/h Luft durch eine entsprechende Erhöhung des Grundlast-Stickstoff ersetzt (wodurch der Vorgang der eigentlichen thermischen Behandlung beendet wurde), die Beheizung des Drehrohres abgeschaltet und das Materialgut durch Einschalten der Schnellkühlung des Drehrohres durch Ansaugen von Umgebungsluft innerhalb von 2 h auf eine unterhalb von 100°C liegende Temperatur und schließlich auf Umgebungstemperatur abgekühlt; der Gasstrom wurde dem Drehrohr dabei mit einer Temperatur von 25°C zugeführt; während der gesamten thermischen Behandlung lag der Druck (unmittelbar) hinter dem Drehrohrausgangs des Gasstroms 0,2 mbar unterhalb des Außendrucks.

Der Sauerstoffgehalt der Gasatmosphäre im Drehrohrofen betrug in allen Phasen der thermischen Behandlung 2,9 Vol.-%. Über die Gesamtdauer der reduktiven thermischen Behandlung arithmetisch gemittelt lag die Ammoniakkonzentration der Gasatmosphäre im Drehrohrofen bei 4 Vol.-%.

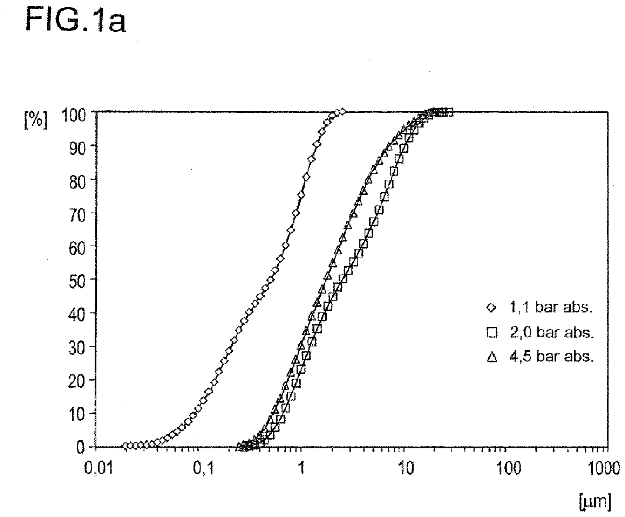

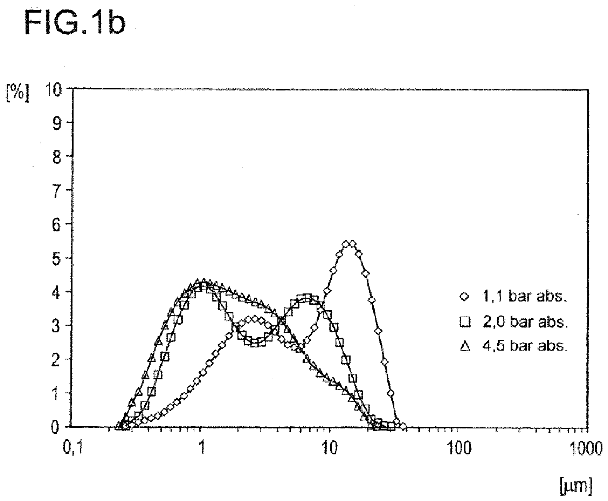

Das erhaltene katalytisch aktive Material wurde mittels einer Biplexquerstromsichtmühle (BQ 500) (Fa. Hosokawa-Aipine Augsburg) zu einem feinteiligen Pulver P gemahlen. Dabei wurden in die Mahlbahnen 24 lange Messer eingebaut. Die Mühlendrehzahl betrug 2500 Umdrehungen/min. Die Ventilatordrosselklappe war ganz geöffnet. Die Dosierung wurde auf 2,5 Umdrehungen/min eingestellt. Der Abluftvolumenstrom betrug 1300 m$^3$/h, der Differenzdruck 10 - 20 mbar. 50 % der Pulverpartikel des aus der Vermahlung resultierenden feinteiligen Pulvers passierten ein Sieb der Maschenweite 1 bis 10 $\mu$m. Der Anteil von Partikeln mit einer Längsausdehnung oberhalb von 50 $\mu$m am feinteiligen Pulver betrug weniger als 1 %. Die Größenverteilung der Partikel des vorstehenden gemahlenen katalytisch aktiven Multielementoxidmassenpulvers zeigen die Figuren 1a und 1b in Abhängigkeit vom Dispergierdruck der zur Trocken-dispergierung verwendeten Druckluft ($\diamond$ = 1,1 bar abs.; $\square$ = 2,0 bar abs.; $\triangle$ = 4,5 bar abs.).

Die der Partikeldurchmesserverteilung der Figuren 1a und 1b zugrunde liegende Messmethode ist die Laserbeugung. Dabei wurde das Multielementoxidmassenpulver über eine Dispergierrinne in den Trockendispergierer Sympatec RODOS (Sympatec GmbH, System-Partikel-Technik, Am Pulverhaus 1, D-38678 Clausthal-Zellerfeld) geführt, dort mit Druckluft (die den jeweiligen Dispergierdruck von 1,1 bzw. 2 bzw. 4,5 bar abs. aufwies) trocken dispergiert und im Freistrahl an die Messzelle geblasen. In dieser wurde dann nach ISO 13320 mit dem Laserbeugungsspektrometer Malvern Mastersizer S (Malvern Instruments, Worcestershire WR14 1AT, United Kingdom) die volumenbezogene Partikeldurchmesserverteilung bestimmt (obscuration 3-7 %).

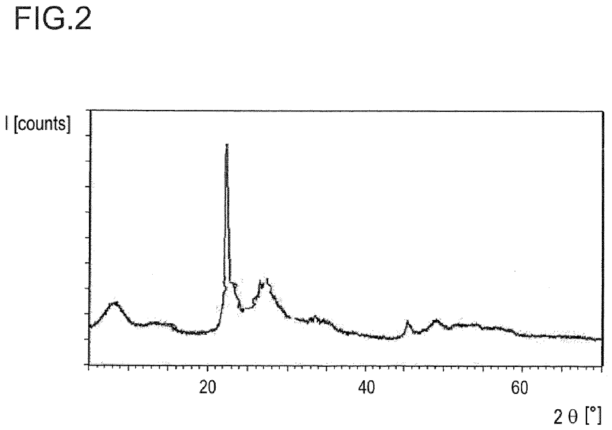

Figur 2 zeigt das Röntgendiffraktogramm des feinteiligen Pulvers P. Die Abszisse zeigt den Beugungswinkel in der 2$\Theta$ Skala [Grad]. Auf der Ordinate ist die absolute Intensität aufgetragen.

## C) Formgebung der Aktivmasse

C1 (Vergleichsbeispiel)

1600 g hohlzylindrische Trägerkörper (7 mm Außendurchmesser, 3 mm Länge, 4 mm Innendurchmesser, Steatit C220 der Fa. CeramTec mit einer Oberflächenrauhigkeit $R_z$ von 45 $\mu$m (Splittauflage)) wurden mit dem gemahlenen feinteiligen Pulver P beschichtet. Die Beschichtung erfolgte in einem HiCoater LHC 25/36 (Fa. Lödige, D-33102 Paderborn). Dieser HiCoater war modifiziert, um eine kontinuierliche Pulverdosierung zu ermöglichen. Diese bestand aus einer trichterförmigen Pulvervorlage, welche über einen Tygon Schlauch (Innendurchmesser: 8 mm, Außendurchmesser 11,1 mm; Fa. Saint-Gobain Performance, 89120 Charny, Frankreich) mit der Trommel des HiCoaters verbunden war. Der Trommelradius beträgt dabei 18 cm. Die Tiefe der Trommel ist 20 cm. Die Achse, um die sich die Trommel drehte, war horizontal ausgerichtet. Für die Beschichtung wurden 750 g des gemahlenen katalytisch aktiven Oxidmassenpulvers in die Pulvervorlage gefüllt. Die Pulverdosierung erfolgte durch kontinuierliche Druckdosierung. Das Pulszeitventil wurde auf 50 ms eingestellt und der eingestellte Druck betrug 0,7 bar über Umgebungsdruck ($\sim$1 atm). Das Pulver in der trichterförmigen Pulvervorlage wurde während der Beschichtung kontinuierlich gerührt, um eine gleichmäßige Dosierung zu gewährleisten (Laufzeit Rührer: 2 s, Pausenzeit Rührer 1 s, modifizierter V-förmiger Ankerrührer, Eigenbau der Fa. BASF SE). Das Bindemittel war eine wässrige Lösung aus 75 Gew.-% Wasser und 25 Gew.-% Glycerin. Dieses wird separat über eine Flüssigkeitsdosierung in die Trommel gesprüht. Die Flüssigkeit wurde mit einer HPLC Pumpe der Fa. Watson-Marlow (Typ 323) in den Dosierarm, der sich in der Trommel befindet, gepumpt (Sprühdruck 3 bar, Formierdruck 2 bar, Mengenstrom: 3 g Glycerin-, Wasserlösung (1:3)/min). Die Pulverdosierung und die Flüssigkeitsdosierung sind parallel zueinander angeordnet. Die am Dosierarm angebrachte Düse der Fa. Schlick (DE) vom Typ 570/0 S75 sowie die ebenfalls am Dosierarm unterhalb befestigte Austrittsöffnung der Feststoffdosierung wurden parallel im Abstand von 6 cm und mit Hilfe eines Winkelmessgerätes im Winkel von 40° zur Waagrechten ausgerichtet. Die Pulverdosierung erfolgt außerhalb des Sprühkegels der Düse. Die Düsen- und Austrittsöffnung der Feststoffdosierung zeigen dabei ein Drehrichung der Trommel. Die Trommel rotierte während der Beschichtung mit 15 U/min im Urzeigersinn. Die Beschichtung erfolgte bei 25 °C über einen Zeitraum von 50min. Danach wurde die beschichteten Trägermaterialien 27 min lang bei 130 °C Zulufttemperatur und 81 °C Ablufttemperatur getrocknet. Danach wurden sie in der ruhenden Trommel über einen Zeitraum von 30 min auf 25 °C abgekühlt. Während der Beschichtung wurde das zugeführte Pulver größtenteils auf der Oberfläche des Trägers aufgenommen. Die nicht aufgenommenen Teile wurden in einem Filter nach der Trommel aufgefangen. Es erfolgte keine Ausbildung von Zwillingen und eine Agglomeration der feinteiligen oxidischen Masse wurde nicht beobachtet.

Die beschichteten Trägerformkörper wurden im Umlufttrockenschrank Fa. Memmert GmbH + Co. KG (Typ UM 400; Innenvolumen = 53 l; Luftstrom = 800 l/h) behandelt, um das noch in der Probe vorhandene Glycerin zu entfernen. Dazu wurde der Umlufttrockenschrank in 2 h auf 300 °C (einschließlich der Lufttemperatur) aufgeheizt und dann für 2 h bei 300 °C gehalten. Das Trockengut befand sich während der Trocknung auf einem im Trockenschrank zentriert platzierten Lochblech (der Lochdurchmesser der über das Lochblech gleichmäßig verteilten Durchtrittsöffnungen = 0,5 cm; das Öffnungsverhältnis des Lochblechs war 60%; die Gesamtquerschnittsfläche des Lochblechs war 35 cm x 26 cm = 910 cm$^2$) geschichtet (Schichthöhe = 2 cm). Danach wurde der Umlufttrockenschrank innerhalb von 2 bis 3 h auf 40 bis 50 °C abgekühlt und die Probe wurde entnommen. Die dem Umlufttrockenschrank entnommenen hohlzylindrischen Schalenkatalysatoren C1 wiesen, bezogen auf ihre Gesamtmasse, einen oxidischen Schalenanteil von 29,1 Gew.-% auf.

C2 (Vergleichsbeispiel)

335 kg des gemahlenen feinteiligen Pulvers P wurden mit 50 kg Molybdäntrioxid der Fa. Climax (66,6 Gew.% Mo, das $MoO_3$ erfüllt sämtliche in der DE 10 2007 010 422 A1 aufgeführten Anforderungen) in einem Mischer vom Typ R645 der Fa. AMK in Aachen (DE) bei einer Mischzeit von 10 min intensiv vermischt. Dabei handelt es sich um einen Schräglagenmischer mit Schneidflügel (Intensivmischer). Der Mischarm dreht sich mit 39 Umdrehungen/min. Das resultierende Pulver wird im Folgenden als Pulver PMo bezeichnet.

Die Formgebung erfolgte nun wie folgt: 61 kg hohlzylindrische Trägerkörper (7 mm Außendurchmesser, 3 mm Länge, 4 mm Innendurchmesser; Steatit des Typs C220 der Fa. Ceram Tec mit einer Oberflächenrauhigkeit Rz von 45 $\mu$m und einem auf das Volumen des Trägerkörpers bezogenen Porengesamtvolumens von ≤1 Vol.-%; vgl. DE-A 2135620) wurden in einen Dragierkessel (Neigungswinkel 90°; Hicoater der Fa. Lödige, DE) von 200 l Innenvolumen gefüllt. Anschließend wurde der Dragierkessel mit 16 U/min in Rotation versetzt. Über eine Düse vom Typ Schlick 0,5 mm, 90° wurden innerhalb von 40 min 3,8 bis 4,2 Liter einer wässrigen Lösung aus 75 Gew.-% Wasser und 25 Gew.-% Glycerin bei einem Flüssigkeitsvordruck von ca. 1,8 bar auf die Trägerkörper aufgesprüht. Gleichzeitig wurden im selben Zeitraum 18,3 kg des Pulvers PMo (über eine Schüttelrinne außerhalb des Sprühkegels der Zerstäuberdüse kontinuierlich zudosiert. Während der Beschichtung wurde das zugeführte Pulver vollständig auf die Oberfläche des Trägerkörpers aufgenommen, eine Agglomeration der feinteiligen oxidischen Aktivmasse oder Zwillingsbildung wurde nicht beobachtet.

Nach beendeter Zugabe von Aktivmassenpulver und wässriger Lösung wurde bei einer Drehgeschwindigkeit von 2 U/min 40 min (alternativ 15 bis 60 min) 110°C (alternativ 80 bis 120°C) heiße Luft (ca. 400 m$^3$/h) in den Dragierkessel geblasen.

Es wurde eine Probe von etwa 2 kg beschichtetem Aktivmassenpulver genommen. Das noch in der Probe vorhandene Glycerin wurde im Umlufttrockenschrank der Fa. Memmert GmbH + Co. KG (Typ UM 400; Innenvolumen = 53 l; Luftstrom = 800 l/h) entfernt. Die Wärmebehandlungsbedingungen waren identisch mit denen des Beispiels C1. Die dem Umlufttrockenschrank entnommenen hohlzylindrischen Schalenkatalysatoren C2 wiesen, bezogen auf ihre Gesamtmasse, einen oxidischen Schalenanteil von 22 Gew.-% auf.

C3 (Vergleichsbeispiel)

Die Formgebung erfolgte nun wie folgt: 70 kg hohlzylindrische Trägerkörper (7 mm Außendurchmesser, 3mm Länge, 4mm Innendurchmesser; Steatit des Typs C220 der Fa. Ceram Tec mit einer Oberflächenrauhigkeit Rz von 45 $\mu$m und einem auf das Volumen des Trägerkörpers bezogenen Porengesamtvolumens von ≤1 Vol.-%; vgl. DE-A 2135620) wurden in einen Dragierkessel (Neigungswinkel 90°; Hicoater der Fa. Lödige, DE) von 200 l Innenvolumen gefüllt. Anschließend wurde der Dragierkessel mit 16 U/min in Rotation versetzt. Über eine Düse vom Typ Schlick 0,5 mm, 90° wurden innerhalb von 40 min 3,8 bis 4,2 Liter einer wässrigen Lösung aus 75 Gew.-% Wasser und 25 Gew.-% Glycerin bei einem Flüssigkeitsvordruck von ca. 1,8 bar auf die Trägerkörper aufgesprüht. Gleichzeitig wurden im selben Zeitraum 18,2 kg des gemahlenen feinteiligen Pulvers P (dessen spezifische Oberfläche 16 m$^2$/g betrug) über eine Schüttelrinne außerhalb des Sprühkegels der Zerstäuberdüse kontinuierlich zudosiert. Während der Beschichtung wurde das zugeführte Pulver vollständig auf die Oberfläche des Trägerkörpers aufgenommen, eine Agglomeration der feinteiligen oxidischen Aktivmasse oder Zwillingsbildung wurde nicht beobachtet.

Nach beendeter Zugabe von Aktivmassenpulver und Wasser wurde bei einer Drehgeschwindigkeit von 2 U/min 40 min (alternativ 15 bis 60 min) 110°C (alternativ 80 bis 120°C) heiße Luft (ca. 400 m$^3$/h) in den Dragierkessel geblasen. Es wurden hohlzylindrische Schalenkatalysatoren erhalten, deren Anteil an oxidischer Aktivmasse, bezogen auf die Gesamtmasse, 20 Gew.-% betrug.

Es wurde eine Probe von etwa 2 kg beschichtetem Aktivmassenpulver genommen. Das noch in der Probe vorhandene Glycerin wurde im Umlufttrockenschrank der Fa. Memmert GmbH + Co. KG (Typ UM 400; Innenvolumen = 53 l; Luftstrom = 800 l/h) entfernt. Die Wärmebehandlungsbedingungen waren identisch mit denen des Beispiels C1. Die dem Umlufttrockenschrank entnommenen hohlzylindrischen Schalenkatalysatoren C3 wiesen, bezogen auf ihre Gesamtmasse, einen oxidischen Schalenanteil von 20 Gew.-% auf.

C4 (Vergleichsbeispiel)

Die Formgebung des Katalysators C4 erfolgte wie bei C1, wobei jedoch im Unterschied zu C1 nur 600 g Pulver in die Pulvervorlage gefüllt wurden und die Beschichtung über einen Zeitraum von 40 min erfolgte. Nach der wie bei C1 durchgeführten Wärmebehandlung im Umlufttrockenschrank wiesen die dem Umlufttrockenschrank entnommenen hohlzylindrischen Schalenkatalysatoren C4, bezogen auf ihre Gesamtmasse, einen oxidischen Schalenanteil von 20,0 Gew.-% auf.

C5 (Vergleichsbeispiel)

Die Beschichtung erfolgte in einer rotierenden Dragiertrommel (Innendurchmesser = 25,5 cm; 36 U/min) mit einem Granulierwerk der Fa. ERWEKA (DE). Die Drehachse der Trommel war dabei in einem Winkel von 51,6° in Bezug auf die Waagrechte angestellt. Die Dragiertrommel wurde mit 800 g von hohlzylindrischen Trägerkörpern (7 mm Außendurchmesser, 3 mm Länge, 4 mm Innendurchmesser, Steatit C220 der Fa. CeramTec mit einer Oberflächenrauhigkeit Rz von 45 $\mu$m (Splittauflage)) befüllt. Als Bindemittel wurde eine wässrige Lösung aus 75 Gew.-% Wasser und 25 Gew.-% Glycerin eingesetzt. Etwa 76 g des flüssigen Bindemittels wurden über eine Düse (Düsendurchmesser = 1 mm) innerhalb der Beschichtungszeit von 45 min auf die Trägerkörper aufgesprüht. Gleichzeitig wurden im selben Zeitraum 200 g gemahlenen feinteiligen Pulver P mittels einer Förderschnecke außerhalb des Sprühkegels der Zerstäuberdüse kontinuierlich zudosiert. Während der Beschichtung wurde das zugeführte Pulver vollständig auf die Oberfläche der Trägerkörper aufgenommen. Eine Agglomeration der feinteiligen oxidischen Aktivmasse wurde nicht beobachtet. Die Beschichung wurde wiederholt. Die gesamte Menge der beiden Beschichtungsversuche wurde zu einer Probe zusammengefasst. Die Probe wurde im Umlufttrockenschrank Fa. Memmert GmbH + Co. KG (Typ UM 400; Innenvolumen = 53 l; Luftstrom = 800 l/h) behandelt, um das noch in der Probe vorhandene Glycerin zu entfernen. Die Wärmebehandlungsbedingungen waren identisch mit denen des Beispiels C1. Die dem Umlufttrockenschrank entnommenen hohlzylindrischen Schalenkatalysatoren C5 wiesen, bezogen auf ihre Gesamtmasse, einen oxidischen Schalenanteil von 19,6 Gew.-% auf.

C6 (Vergleichsbeispiel)

Die Formgebung des Katalysators C6 erfolgte wie bei C1, wobei jedoch im Unterschied zu C1 nur 451 g Pulver in die Pulvervorlage gefüllt wurden und die Beschichtung über einen Zeitraum von 30 min erfolgte. Nach der wie bei C1 durchgeführten Wärmebehandlung im Umlufttrockenschrank wiesen die dem Umlufttrockenschrank entnommenen hohlzylindrischen Schalenkatalysatoren C6, bezogen auf ihre Gesamtmasse, einen oxidischen Schalenanteil von 18,0 Gew.-% auf.

C7 (Vergleichsbeispiel)

Die Formgebung des Katalysators C7 erfolgte wie bei C1, wobei jedoch im Unterschied zu C1 nur 377,5 g Pulver in die Pulvervorlage gefüllt wurden und die Beschichtung über einen Zeitraum von 25 min erfolgte. Nach der wie bei C1 durchgeführten Wärmebehandlung im Umlufttrockenschrank wiesen die dem Umlufttrockenschrank entnommenen hohlzylindrischen Schalenkatalysatoren C7, bezogen auf ihre Gesamtmasse, einen oxidischen Schalenanteil von 15,8 Gew.-% auf.

C8 (Vergleichsbeispiel)

Die Formgebung des Katalysators C8 erfolgte wie bei C5, wobei jedoch im Unterschied zu C5 nur etwa 44 g des flüssigen Bindemittels über eine Düse (Düsendurchmesser = 1 mm) auf die Trägerkörper aufgesprüht wurde und die Beschichtung innerhalb von 27,5 min erfolgte. Nach der wie bei C1 durchgeführten Wärmebehandlung im Umlufttrockenschrank wiesen die dem Umlufttrockenschrank entnommenen hohlzylindrischen Schalenkatalysatoren C8, bezogen auf ihre Gesamtmasse, einen oxidischen Schalenanteil von 15,5 Gew.-% auf.

C9 (Beispiel)

Die Formgebung des Katalysators C9 erfolgte wie bei C5, wobei jedoch im Unterschied zu C5 nur etwa 29 g des flüssigen Bindemittels über eine Düse (Düsendurchmesser = 1 mm) auf die Trägerkörper aufgesprüht wurde und die Beschichtung innerhalb von 16 min erfolgte. Nach der wie bei C1 durchgeführten Wärmebehandlung im Umlufttrockenschrank wiesen die dem Umlufttrockenschrank entnommenen hohlzylindrischen Schalenkatalysatoren C9, bezogen auf ihre Gesamtmasse, einen oxidischen Schalenanteil von 10,8 Gew.-% auf.

C10 (Beispiel)

Die Formgebung des Katalysators C10 erfolgte wie bei C1, wobei jedoch im Unterschied zu C1 nur 300 g Pulver in die Pulvervorlage gefüllt wurden und die Beschichtung über einen Zeitraum von 20 min erfolgte. Nach der wie bei C1 durchgeführten Wärmebehandlung im Umlufttrockenschrank wiesen die dem Umlufttrockenschrank entnommenen hohlzylindrischen Schalenkatalysatoren C10, bezogen auf ihre Gesamtmasse, einen oxidischen Schalenanteil von 10,4 Gew.-% auf.

C11 (Beispiel)

Wie im Beispiel C2 wurde das feinteilige Pulver P mit $MoO_3$ vermischt. Im Unterschied zum Beispiel C2 wurden jedoch nur etwa 1,9 - 2,1 Liter der wässrigen Lösung (Glycerin/Wasser=1/3) innerhalb von etwa 20 min auf die Trägerkörper aufgesprüht. Gleichzeitig wurden im selben Zeitraum nur etwa 8 kg des Pulver PMo kontinuierlich zudosiert. Nach der Beschichtung erfolgte wie im Beispiel C2 eine Wärmebehandlung in der Beschichtungsapparatur.

Es wurde eine Probe von etwa 2 kg beschichtetem Aktivmassenpulver genommen. Das noch in der Probe vorhandene Glycerin wurde im Umlufttrockenschrank der Fa. Memmert GmbH + Co. KG (Typ UM 400; Innenvolumen = 53 l; Luftstrom = 800 l/h) entfernt. Die Wärmebehandlungsbedingungen waren identisch mit denen des Beispiels C1. Die dem Umlufttrockenschrank entnommenen hohlzylindrischen Schalenkatalysatoren C11 wiesen, bezogen auf ihre Gesamtmasse, einen oxidischen Schalenanteil von 10 Gew.-% auf.

C12 (Vergleichsbeispiel)

Die Formgebung des Katalysators C12 erfolgte wie bei C1, wobei jedoch im Unterschied zu C1 nur 345,5 g Pulver in die Pulvervorlage gefüllt wurden und die Beschichtung über einen Zeitraum von 23 min erfolgte. Nach der wie bei C1 durchgeführten Wärmebehandlung im Umlufttrockenschrank wiesen die dem Umlufttrockenschrank entnommenen hohlzylindrischen Schalenkatalysatoren C12, bezogen auf ihre Gesamtmasse, einen oxidischen Schalenanteil von 14 Gew.-% auf. Die Aktivmassebedeckung des Schalenkatalysators C12 beträgt 0,23 mg/mm$^2$.

C13 (Beispiel)

Die Formgebung des Katalysators C13 erfolgte wie bei C1, wobei jedoch im Unterschied zu C1 nur 360,7 g Pulver in die Pulvervorlage gefüllt wurden und die Beschichtung über einen Zeitraum von 24 min erfolgte. Nach der wie bei C1 durchgeführten Wärmebehandlung im Umlufttrockenschrank wiesen die dem Umlufttrockenschrank entnommenen hohlzylindrischen Schalenkatalysatoren C13, bezogen auf ihre Gesamtmasse, einen oxidischen Schalenanteil von 13,4 Gew.-% auf.

Die Eigenschaften der Katalysatoren gemäß Beispiel C1 bis C11 sind in Tabelle 1 dargestellt. Die spezifische geometrische Oberfläche $S_m$ des Trägerformkörpers betrug in allen Beispielen 0,725 mm$^2$/mg. Sie wurde berechnet, indem man die geometrische Oberfläche eines Trägerformkörpers (155,5 mm$^2$) durch dessen Masse (214,4 mg) teilte.

Tabelle 1:

| Beispiel | Q[1] [Gew.-%] | q[2] [mg/mm$^2$] | $V_{0,02-6,5}$[3] | $V_{0,26-2}$[4] | $p_{vol}$[5] | $t_B$[6] [min] |
|---|---|---|---|---|---|---|
| | | | [ml/(g Aktivmasse)] | | | |
| C1* | 29,1 | 0,57 | 0,302 | 0,154 | 0,51 | 50 |
| C2* | 22,0 | 0,39 | 0,163 | 0,024 | 0,15 | 40 |
| C3* | 20,0 | 0,34 | 0,205 | 0,054 | 0,26 | 40 |
| C4* | 20,0 | 0,34 | 0,243 | 0,084 | 0,34 | 40 |
| C5* | 19,6 | 0,34 | 0,246 | 0,063 | 0,26 | 90 |
| C6* | 18,0 | 0,30 | 0,335 | 0,187 | 0,56 | 30 |
| C7* | 15,8 | 0,26 | 0,270 | 0,133 | 0,49 | 25 |
| C8* | 15,5 | 0,25 | 0,321 | 0,129 | 0,40 | 55 |
| C9 | 10,8 | 0,17 | 0,247 | 0,092 | 0,37 | 32 |
| C10 | 10,4 | 0,16 | 0,307 | 0,153 | 0,50 | 20 |
| C11 | 10,0 | 0,15 | 0,188 | 0,045 | 0,24 | 25 |

*: nicht erfindungsgemäß
[1] Aktivmasseanteil des Katalysators
[2] Aktivmassebedeckung
[3] Volumen der Poren mit mittlerem Durchmessern im Bereich von 0,26 bis 2 μm
[4] Volumen der Poren mit mittlerem Durchmessern im Bereich von 0,02 bis 6,5 μm
[5] Volumenanteil an Makroporen mit mittlerem Durchmessern im Bereich von 0,26 bis 2 μm im Verhältnis zum Gesamtporenvolumen im Bereich von 0,02 bis 6,5 μm
[6] Dauer des Beschichtungsvorgangs

In dieser Schrift beziehen sich alle Angaben zur Porenbeschaffenheit von festen Stoffen, soweit nichts anderes explizit erwähnt wird, auf Bestimmungen mit der Methode der Quecksilberporosimetrie in Anwendung eines Gerätes Auto Pore IV 9520 der Firma Micromeritics in Norcross, Georgia 30093-1877, USA. Im Fall von untersuchten Pulvern betrug die in den Probenraum jeweils eingebrachte Probenmenge 2,5 g. Im Fall von untersuchten Schalenkatalysatoren wurden jeweils 5 Stücke des jeweiligen Schalenkatalysators in den Probenraum eingebracht (der Beitrag der Poren des geometrischen Trägerformkörpers des Schalenkatalysators war dabei in den untersuchten Fällen im Vergleich zum Beitrag der Poren der Aktivmassenschale vernachlässigbar).

Der Probenraum war in eine langgezogene Kapillare fortgeführt, sodass geringen Druckänderungen deutliche Änderungen der Länge des in die Kapillare ragenden Quecksilberfadens entsprachen. Das genutzte Kapillarvolumen lag in allen Fällen zwischen 25 und 91 Vol.-%, bezogen auf das Kapillargesamtvolumen.

Vor Beginn einer jeweiligen Probenuntersuchung wurde der Probenraum (bei 25 °C) jeweils bis zu einem Innendruck von 9,3 x 10$^{-4}$ bar evakuiert und die Probe während 20 Minuten bei dieser Temperatur und bei diesem Druck entgast. Danach wurde das Quecksilber mit über die Zeit ansteigenden Drucken bis zu einem Enddruck von 4137 bar in den Probenraum gedrückt. Der Anfangsdruck betrug 0,04 bar. Dem entspricht eine (Band)Breite an erfassten Porendurchmessern von 0,003 $\mu$m bis 360 $\mu$m.

Figur 3 der vorliegenden Schrift zeigt die Porendurchmesserverteilung der Poren der Aktivmassenschale von C1. Auf der Abszisse ist der jeweilige Porendurchmesser in $\mu$m aufgetragen (logarithmische Auftragung zur Basis 10). Auf der linken Ordinate ist in ([ml]/[g Aktivmasse]) das Integral über die individuellen Beiträge der einzelnen Porendurchmesser zum spezifischen Porengesamtvolumen (der kumulative Beitrag zum vorgenannten spezifischen Porengesamtvolumen) aufgetragen (Kurve □). Der Endpunkt ist das auf die Aktivmasse bezogene (spezifische) Porengesamtvolumen (Gesamtintrusionsvolumen).

**Gasphasenoxidation von Acrolein zu Acrylsäure**

Ein Reaktionsrohr (V2A Stahl; 30 mm Außendurchmesser; 2 mm Wandstärke; 26 mm Innendurchmesser; 464 cm Länge) enthielt, von oben nach unten:

Abschnitt 1:    79 cm Länge, Leerrohr;

Abschnitt 2:    62 cm Länge, Vorschüttung aus Steatit-Ringen der Geometrie 7 mm x 3 mm x 4 mm (Außendurchmesser x Länge x Innendurchmesser; Steatit C 220 der Fa. CeramTec);

Abschnitt 3:    100 cm Länge, Katalysatorfestbett aus einem homogenen Gemisch bestehend aus 20 Gew.-% Steatit-Ringen der Geometrie 7 mm x 3 mm x 4 mm (Aussendurchmesser x Länge x Innendurchmesser; Steatit C 220 der Fa. CeramTec) und 80 Gew.-% des jeweiligen Katalysators;

Abschnitt 4:    200 cm Länge, Katalysatorfestbett ausschließlich bestehend aus dem in Abschnitt 3 jeweils verwendeten Katalysator;

Abschnitt 5:    10 cm Länge, Nachschüttung aus denselben Steatit-Ringen wie in Abschnitt 2;

Abschnitt 6:    14 cm Länge, Katalysatorstuhl aus V2A Stahl zur Aufnahme des Katalysatorfestbetts.

Durch das jeweilige Reaktionsrohr wurde von oben nach unten durch das Reaktionsrohr strömend ein Reaktionsgasgemisch geführt, das bei Eintritt in das Reaktionsrohr folgende Gehalte aufwies:

4,3 Vol.-% Acrolein,
0,2 Vol.-% Propen,
0,2 Vol.-% Propan,
0,3 Vol.-% Acrylsäure,
5,4 Vol.-% $O_2$,
7,0 Vol.-% $H_2O$,
0,4 Vol.-% CO und $CO_2$
Rest $N_2$.

Die Belastung des Katalysatorfestbetts mit Acrolein betrug jeweils 75 Nl/(lh).

Das Reaktionsrohr war über seine Länge (bis auf die letzten 10 cm des Leerrohres im Abschnitt 1 und die letzten 3 cm des Rohres im Abschnitt 6) jeweils von einem gerührten und von außen elektrisch beheizten Salzbad (Gemisch aus 53 Gew.-% Kaliumnitrat, 40 Gew.-% Natriumnitrit und 7 Gew.-% Natriumnitrat, 50 kg Salzschmelze) umspült (die Strömungsgeschwindigkeit am Rohr war 3 m/s (in der Ebene senkrecht zur Längsachse des Rohres)).

Die Salzbadtemperatur $T^B$ entspricht der Temperatur, mit der die Salzschmelze in das Salzbad geführt wurde. Sie wurde in allen Fällen so eingestellt, dass ein auf den einfachen Durchgang des Reaktionsgemischs durch das Katalysatorfestbett bezogener Acroleinumsatz $U^A$ von 99,3 mol-% resultierte. Entlang des Reaktionsrohres änderte sich die Temperatur des Salzbades infolge Zuheizung nicht (es wurde vom Salzbad mehr Wärme abgestrahlt als vom Reaktionsrohr an das Salzbad abgegeben wurde). Am Eingang des Reaktionsrohres entsprach die Temperatur des Reaktionsgases der jeweiligen Salzbadtemperatur $T^B$. Die höchste lokale Temperatur $T^H$ wurde durch eine Punktmessung in dem Reaktionsrohr bestimmt. Die unter Verwendung verschiedener Katalysatoren erzielten Ergebnisse sind in Tabelle 2 zusammengefasst.

Unter der Selektivität der Acrylsäurebildung ($S^{AS}$ (mol-%)) wird in dieser Schrift verstanden:

$$S^{AS} = \frac{\text{Molzahl Acrolein umgesetzt zu Acrylsäure}}{\text{Molzahl Acrolein insgesamt umgesetzt}} \times 100.$$

(die Umsatzzahlen jeweils bezogen auf einen einmaligen Durchgang des Reaktionsgasgemischs durch das Katalysatorfestbett).

Die folgende Tabelle 2 zeigt die in Abhängigkeit vom eingesetzten Schalenkatalysator nach jeweils 100 Betriebsstunden resultierenden Ergebnisse:

Tabelle 2

| Beispiel | $T^B$ [°C] | $T^H$ [°C] | $S^{AS\ 1)}$ [mol%] |
|---|---|---|---|
| C1* | 245 | 267 | 96,7 |
| C2* | 260 | 279 | 97,0 |
| C3* | 260 | 277 | 97,1 |
| C4* | 258 | 278 | 97,1 |
| C5* | 259 | 277 | 97,2 |
| C6* | 259 | 281 | 97,7 |
| C7* | 262 | 284 | 97,7 |
| C8* | 264 | 286 | 97,5 |
| C9 | 272 | 292 | 97,7 |
| C10 | 279 | 299 | 97,9 |
| C11 | 275 | 296 | 97,6 |
| *: nicht erfindungsgemäß<br>1) Selektivität der Acrylsäurebildung | | | |

Bei einer Aktivmassebedeckung des Katalysators von höchstens 0,3 mg/mm² beträgt $S^{AS}$ 97,6 mol% oder mehr. Bei höherer Aktivmassebedeckung des Katalysators ist $S^{AS}$ mit Werten von 96,7 bis 97,2 mol% geringer. Der Vorteil der erfindungsgemäßen Aktivmassebedeckung stellt sich trotz höherer Heißpunkttemperaturen $T^H$ von 281-299°C ein.

Der Vorteil des erfindungsgemäß bevorzugten hohen Volumenanteils an Makroporen ($p_{vol}$) zeigt sich beim Vergleich der Ergebnisse, die mit Katalysatoren mit im Wesentlichen gleicher Aktivmassebedeckung erzielt wurden. In der Gruppe der Katalysatoren mit Aktivmassebedeckung von 0,15 bis 0,17 mg/mm² ist bei $p_{vol}$ = 0,5 $S^{AS}$ besonders hoch (97,9 mol%, C10), während bei $p_{vol}$ = 0,24 $S^{AS}$ erniedrigt ist (97,6 mol%, C11). Unter den beiden Katalysatoren mit Aktivmassebedeckung im Bereich von 0,25 bis 0,26 mg/mm² ist bei $p_{vol}$ = 0,49 $S^{AS}$ hoch (97,7 mol%, C7), während bei $p_{vol}$ = 0,40 $S^{AS}$ erniedrigt ist (97,5 mol%, C8).

**Gasphasenoxidation von Acrolein zu Acrylsäure unter Verwendung eines Katalysatorfestbetts mit zwei aufeinanderfolgenden Reaktionszonen**

Ein Reaktionsrohr (Edelstahl Typ 1.4541 (EU Normnummer EN 10088-3; 33,7 mm Außendurchmesser; 2 mm Wandstärke; 29,7 mm Innendurchmesser; 400 cm Länge, 4 mm Thermohülse) wurde von unten nach oben wie folgt beschickt:

Abschnitt 1:    70 cm Länge
Vorschüttung aus Steatit-Ringen der Geometrie 7 mm x 3 mm x 4 mm (Außendurchmesser x Länge x Innendurchmesser; Steatit C220 der Fa. CeramTec);

Abschnitt 2:    100 cm Länge
Katalysatorfestbettschüttung aus dem jeweiligen Schalenkatalysators;

Abschnitt 3:    200 cm Länge
Katalysatorfestbettschüttung aus dem jeweiligen Schalenkatalysator;

Abschnitt 4:    8 cm Länge
Nachschüttung aus denselben Steatit-Ringen wie in Abschnitt 1;

Abschnitt 5:    23 cm Länge
Leerrohr

Durch das jeweilige wie vorstehend beschrieben beschickte Reaktionsrohr wurde von oben nach unten durch das Reaktionsrohr strömend ein Reaktionsgasgemisch geführt, das folgende Gehalte aufwies:

| | | |
|---|---|---|
| 4,5 | Vol.-% | Acrolein, |
| 0,1 | Vol.-% | Propen, |
| 0,07 | Vol.-% | Propan, |
| 0,5 | Vol.-% | Acrylsäure, |
| 5,4 | Vol.-% | $O_2$, |
| 7 | Vol.-% | $H_2O$, |
| 0,6 | Vol.-% | CO und CO2, und |
| Rest | $N_2$. | |

Die Belastung des Katalysatorfestbetts (wie in der DE-A 19927624 definiert) mit Acrolein betrug jeweils 75 Nl/(lh).

Das Reaktionsrohr war über seine Länge jeweils von einem gerührten und von außen elektrisch beheizten Salzbad (Gemisch aus 53 Gew.-% Kaliumnitrat, 40 Gew.-% Natriumnitrit und 7 Gew.-% Natriumnitrat; 50 kg Salzschmelze) umspült (die Strömungsgeschwindigkeit am Rohr war 3 m/s (in der Ebene senkrecht zur Längsachse des Rohres)).

Die Salzbadtemperatur $T^B$ (°C) (mit der das Salzbad zugeführt wurde) wurde in allen Fällen so eingestellt, dass ein auf den einfachen Durchgang des Reaktionsgasgemischs durch das Katalysatorfestbett bezogener Acroleinumsatz von 98,3 mol-% resultierte. Entlang des Reaktionsrohres änderte sich die Salzbadtemperatur infolge Zuheizung nicht (es wurde vom Salzbad mehr Wärme abgestrahlt wie vom Reaktionsrohr an das Salzbad abgegeben). Die Zuführtemperatur des Reaktionsgasgemischs (am Eingang in das Reaktionsrohr) war jeweils auf die jeweilige Salzbadtemperatur eingestellt.

Die Temperatur im Katalysatorbett wurde durch ein Thermoelement, welches in einer Thermohülse, die sich im Inneren des Reaktorrohres befand, platziert war und mit Hilfe einer Zugmaschine von unten nach oben im Reaktorbett geschoben wurde, kontinuierlich gemessen. Die maximale Temperatur dieser Messung entsprach der Heißpunkttemperatur $T^H$.

Die nachfolgende Tabelle 3 zeigt die nach 100 Betriebsstunden resultierenden Ergebnisse, die sich je nach Beschickung des Reaktorabschnitts 2 und 3 mit unterschiedlichen erfindungsgemäßen und nicht erfindungsgemäßen Schalenkatalysatoren einstellt.

Tabelle 3

| | Reaktorbefüllung | | $T^B$ | $T^H$ | $x^{H***}$ | $S^{AS}$ |
|---|---|---|---|---|---|---|
| | Abschnitt 2 | Abschnitt 3 | [°C] | [°C] | [cm] | [mol %] |
| Vergleichsbeispiel | 70 Gew.% C3*, 30 Gew.% Steatitringe** | 100 Gew.% C3* | 263 | 312 | 61 | 97,0 |
| D1 | 100 Gew.% C10 | 100 Gew.% C8* | 264 | 309 | 74 | 98,3 |
| D2 | 100 Gew.% C12 | 100 Gew.% C3* | 261 | 313 | 81 | 97,5 |
| *: nicht erfindungsgemäß<br>**: Steatitringe der Geometrie 7 mm x 3 mm x 4 mm (Steatit C220 der Fa. CeramTec)<br>***: Abstand des in Abschnitt 2 befindlichen Heißpunkts vom Übergang des Abschnitts 1 zum Abschnitt 2 | | | | | | |

Der Vergleich von Beispiel D2 mit dem Vergleichsbeispiel in Tabelle 3 weist durch die bessere Acrylsäureselektivität (97,5 mol%) von D2 im Vergleich zur Referenz (97 mol%) aus, dass die Befüllung derjenigen Reaktionszone mit der höchsten Temperatur (der Heißpunkt befindet sich in D2 und der Referenz in Abschnitt 2, siehe Tabelle 3) mit erfindungsgemäßen Schalenkatalysatoren im Hinblick auf die Selektivität der Acrylsäurebildung vorteilhaft ist.

## Teilwechsel der stromaufwärts gelegenen Reaktionszone eines Katalysatorfestbetts

Die Deutsche Patentanmeldung mit dem Aktenzeichen 102 32 748 beschreibt den Austausch einer Teilmenge des Katalysatorfestbetts durch eine frische Katalysatorbeschickung (siehe Akte der Europäischen Patentanmeldung mit der Veröffentlichungsnummer EP 1 525 178 A0, welche deren Priorität beansprucht). Im Folgenden wurde untersucht, ob die erfindungsgemäßen Katalysatoren auch für den in dieser Deutschen Patentanmeldung beschriebenen Teilwechsel eines Katalysatorfestbetts vorteilhaft sind.

Ein Reaktionsrohr (Edelstahl Typ 1.4541 (EU Normnummer EN 10088-3; 33,7 mm Außendurchmesser; 2 mm Wandstärke; 29,7 mm Innendurchmesser; 400 cm Länge, 4 mm Thermohülse) wurde von unten nach oben wie folgt beschickt:

Abschnitt 1: 75 cm Länge
Vorschüttung aus Steatit-Ringen der Geometrie 7 mm x 3 mm x 4 mm (Außendurchmesser x Länge x Innendurchmesser; Steatit C220 der Fa. CeramTec);

Abschnitt 2: 110 cm Länge
Katalysatorfestbettschüttung aus dem jeweiligen Schalenkatalysators;

Abschnitt 3: 190 cm Länge
Katalysatorfestbettschüttung bestehend aus Schalenkatalysator (71 Gew.%) hergestellt gemäß Ausführungsbeispiel 1 der DE 103 60 057 A1 und 29 Gew.% Steatit-Ringen der Geometrie 7 mm x 3 mm x 4 mm (Außendurchmesser x Länge x Innendurchmesser; Steatit C220 der Fa. CeramTec);

Abschnitt 4: 3 cm Länge
Nachschüttung aus denselben Steatit-Ringen wie in Abschnitt 1;

Abschnitt 5: 23 cm Länge
Leerrohr

Durch das jeweilige wie vorstehend beschrieben beschickte Reaktionsrohr wurde von oben nach unten durch das Reaktionsrohr strömend ein Reaktionsgasgemisch geführt, das folgende Gehalte aufwies:

| | | |
|---|---|---|
| 4,5 | Vol.-% | Acrolein, |
| 0,1 | Vol.-% | Propen, |
| 0,07 | Vol.-% | Propan, |
| 0,5 | Vol.-% | Acrylsäure, |
| 5,4 | Vol.-% | $O_2$, |
| 7 | Vol.-% | $H_2O$, |

(fortgesetzt)

| 0,6 | Vol.-% | CO und $CO_2$, und |
|---|---|---|
| Rest | $N_2$. | |

Die Belastung des Katalysatorfestbetts (wie in der DE-A 19927624 definiert) mit Acrolein betrug jeweils 75 Nl/(lh).

Das Reaktionsrohr war über seine Länge jeweils von einem gerührten und von außen elektrisch beheizten Salzbad (Gemisch aus 53 Gew.-% Kaliumnitrat, 40 Gew.-% Natriumnitrit und 7 Gew.-% Natriumnitrat; 50 kg Salzschmelze) umspült (die Strömungsgeschwindigkeit am Rohr war 3 m/s (in der Ebene senkrecht zur Längsachse des Rohres)).

Die Salzbadtemperatur $T^B$ (°C) (mit der das Salzbad zugeführt wurde) wurde in allen Fällen so eingestellt, dass ein auf den einfachen Durchgang des Reaktionsgasgemischs durch das Katalysatorfestbett bezogener Acroleinumsatz von 99,3 mol-% resultierte. Entlang des Reaktionsrohres änderte sich die Salzbadtemperatur infolge Zuheizung nicht (es wurde vom Salzbad mehr Wärme abgestrahlt wie vom Reaktionsrohr an das Salzbad abgegeben). Die Zuführtemperatur des Reaktionsgasgemischs (am Eingang in das Reaktionsrohr) war jeweils auf die jeweilige Salzbadtemperatur eingestellt. Die Temperatur im Katalysatorbett wurde durch ein Thermoelement, welches in einer Thermohülse, die sich im Inneren des Reaktorrohres befand, platziert war und mit Hilfe einer Zugmaschine von unten nach oben im Reaktorbett geschoben wurde, kontinuierlich gemessen. Die maximale Temperatur dieser Messung entsprach der Heißpunkttemperatur $T^H$.

Die nachfolgende Tabelle 4 zeigt die nach 100 Betriebsstunden resultierenden Ergebnisse, die sich je nach Beschickung des Reaktorabschnitts 2 mit erfindungsgemäßen und bzw. nicht erfindungsgemäßen Schalenkatalysatoren einstellt.

Tabelle 4

| | Abschnitt 2 | $T^B$ | $T^H$ | $x^{H***}$ | $S^{AS}$ |
|---|---|---|---|---|---|
| | | [°C] | [°C] | [cm] | [mol %] |
| Vergleichsbeispiel | 50 Gew.% C2*, 50 Gew.% Steatitringe** | 274 | 330 | 46 | 96,9 |
| E1 | 100 Gew.% C11 | 275 | 324 | 71 | 97,5 |
| *: nicht erfindungsgemäß<br>**: Steatitringe der Geometrie 7 mm x 3 mm x 4 mm (Steatit C220 der Fa. CeramTec)<br>***: Abstand des in Abschnitt 2 befindlichen Heißpunkts vom Übergang des Abschnitts 1 zum Abschnitt 2 | | | | | |

Der Vergleich von Beispiel E1 mit dem Vergleichsbeispiel in Tabelle 4 weist durch die bessere Acrylsäureselektivität (97,5 mol%) von Beispiel E1 im Vergleich zur Referenz (96,9 mol%) aus, dass bei einem Katalysatorteilwechsel die Befüllung der vorderen Reaktionszone mit erfindungsgemäßen Schalenkatalysatoren im Hinblick auf die Selektivität der Acrylsäurebildung vorteilhaft ist.

**Gasphasenoxidation von Acrolein zu Acrylsäure unter Verwendung eines Katalysatorfestbetts mit zwei aufeinanderfolgenden Heizzonen**

Ein Reaktionsrohr (Edelstahl Typ 1.4541 (EU Normnummer EN 10088-3, 33,7 mm Außendurchmesser; 2 mm Wandstärke; 29,7 mm Innendurchmesser; 400 cm Länge; 4 mm Thermohülse) wurde von oben nach unten wie folgt beschickt:

Abschnitt 1: 70 cm Länge
Vorschüttung aus Steatit-Ringen der Geometrie 7 mm x 3 mm x 4 mm (Außendurchmesser x Länge x Innendurchmesser; Steatit C220 der Fa. CeramTec)

Abschnitt 2: 100 cm Länge
Katalysatorfestbettschüttung aus dem Schalenkatalysator C13;

Abschnitt 3: 200 cm Länge
Katalysatorfestbettschüttung aus dem Schalenkatalysator C3;

Abschnitt 4:    8 cm Länge
Nachschüttung aus denselben Steatitringen wie in Abschnitt 1;

Abschnitt 5:    23 cm Länge
Leerrohr

Durch das jeweilige wie vorstehend beschrieben beschickte Reaktionsrohr wurde von oben nach unten durch das Reaktionsrohr strömend ein Reaktionsgasgemisch geführt, das die folgenden Gehalte wie aufwies:

| | | |
|---|---|---|
| 4,5 | Vol.-% | Acrolein, |
| 0,1 | Vol.-% | Propen, |
| 0,07 | Vol.-% | Propan, |
| 0,5 | Vol.-% | Acrylsäure, |
| 5,4 | Vol.-% | $O_2$ |
| 7 | Vol.-% | $H_2O$ |
| 0,6 | Vol.-% | CO und $CO_2$, und |
| Rest | $N_2$. | |

Die Belastungen des Katalysatorfestbetts $L^{ACR}$ (wie in der DE-A 19927624 definiert) mit Acrolein betrugen 75, 100 und 145 $Nl_{Acrolein}/(lh)$.

Das Reaktionsrohr wurde mit zwei unterschiedlichen Salzbädern wie in der DE 2010-10048405 beschrieben beheizt. Die ersten 190 cm wurden mittels eines im Gegenstrom gepumpten Salzbades thermostatisiert das mit der Temperatur $T^A$ zugeführt wurde. Die zweiten 210 cm wurden mittels eines im Gegenstrom gepumpten Salzbades B thermostatisiert das mit der Temperatur $T^B$ zugeführt wurde. Beide Salzbäder bestanden aus einem Gemisch aus 53 Gew.-% Kaliumnitrat, 40 Gew.-% Natriumnitrit und 7 Gew.-% Natriumnitrat; 50 kg Salzschmelze. Die Strömungsgeschwindigkeit am Rohr betrug 3 m/s (in der Ebene senkrecht zur Längsachse des Rohres.

Die Salzbadtemperaturen $T^A$ und $T^B$ (°C) (mit der die beiden Salzbäder zugeführt wurden) wurden in allen Fällen so eingestellt, dass ein auf den einfachen Durchgang des Reaktionsgasgemisches durch das Katalysatorbett bezogener Acroleinumsatz von 99,4 mol.-% bei Acroleinbelastungen von 75 und 100 $Nl_{Acrolein}/(lh)$ sowie 98,5 mol.-% bei einer Belastung von 145 $Nl_{Acrolein}/(lh)$ resultierte. Entlang des Reaktionsrohres änderte sich die Salzbadtemperatur infolge Zuheizung nicht (es wurde vom Salzbad mehr Wärme abgestrahlt wie vom Reaktionsrohr an das Salzbad abgegeben wurde). Die Zuführtemperatur des Reaktionsgasgemischs (am Eingang in das Reaktionsrohr) war jeweils auf die jeweilige Salzbadtemperatur eingestellt.

Die Temperatur im Katalysatorbett wurde durch ein Thermoelement, welches in einer Thermohülse, die sich im Inneren des Reaktorrohrs befand, platziert war und mit Hilfe einer Zugmaschine von unten nach oben im Reaktorbett geschoben wurde, kontinuierlich gemessen. Die bei dieser Messung bestimmten Höchsttemperaturen entsprachen den Heißtemperaturen $T^{H1}$ und $T^{H2}$.

Die nachfolgende Tabelle 5 zeigt die nach 100 Betriebsstunden resultierenden Ergebnisse, die sich bei unterschiedlichen Belastungen mit dem erfindungsgemäßen Schalenkatalysator einstellt.

Tabelle 5

| | Reaktorbefüllung | | $L^{ACR}$ | $T^A$ | $T^B$ | $T^{H1}$ | $X^{H1*}$ | $T^{H2}$ | $X^{H2**}$ | $S^{AS}$ |
|---|---|---|---|---|---|---|---|---|---|---|
| | Abschnitt 2 | Abschnitt 3 | [Nl/(lh)] | [°C] | [°C] | [°C] | [cm] | [°C] | [cm] | [mol%] |
| F1 | 100 Gew.-% C13 | 100 % Gew.-% C3 | 75 | 263 | 263 | 314 | 91 | Keine zweite Heißtemperatur gefunden | | 96,5 |
| F2 | 100 % Gew.-% C13 | 100 % Gew.-% C3 | 100 | 265 | 269 | 314 | 95 | 307 | 145 | 96,1 |

(fortgesetzt)

| | Reaktorbefüllung | | $L^{ACR}$ | $T^A$ | $T^B$ | $T^{H1}$ | $X^{H1*}$ | $T^{H2}$ | $X^{H2**}$ | $S^{AS}$ |
|---|---|---|---|---|---|---|---|---|---|---|
| | Abschnitt 2 | Abschnitt 3 | [Nl/ (lh)] | [°C] | [°C] | [°C] | [cm] | [°C] | [cm] | [mol%] |
| F3 | 100 % Gew.-% C13 | 100 % Gew.-% C3 | 145 | 272 | 273 | 320 | 110 | 323 | 135 | 95,8 |

| |
|---|
| *: Abstand des ersten Heißpunkts vom Übergang des Abschnitts 1 zum Abschnitt 2<br>**: Abstand des zweiten Heißpunkts vom Übergang des Abschnitts 1 zum Abschnitt 2 |

**Patentansprüche**

1. Katalysator zur Herstellung einer $\alpha,\beta$-ungesättigten Carbonsäure durch Gasphasenoxidation eines $\alpha,\beta$-ungesättigten Aldehyds, umfassend einen Trägerformkörper mit einer darauf aufgebrachten Aktivmasse, **dadurch gekennzeichnet, dass** die Aktivmassebedeckung q

$$q = \frac{Q}{(100-Q)\, S_m}$$

höchstens 0,22 mg/mm$^2$ beträgt, wobei Q der Aktivmasseanteil des Katalysators in Gew.-% und $S_m$ die spezifische geometrische Oberfläche des Trägerformkörpers in mm$^2$/mg ist und die Aktivmasse eine die Elemente Mo und V enthaltende katalytisch aktive Multielementoxidmasse umfasst, wobei der molare Anteil des Elementes Mo an der Gesamtmenge aller von Sauerstoff verschiedenen Elemente der katalytisch aktiven Multielementoxidmasse 20 mol-% bis 80 mol-% beträgt, und das molare Verhältnis von in der katalytisch aktiven Multielementoxidmasse enthaltenem Mo zu in der katalytisch aktiven Multielementoxidmasse enthaltenem V, Mo/V, 15:1 bis 1:1 beträgt.

2. Katalysator nach Anspruch 1, **dadurch gekennzeichnet, dass** der Volumenanteil $p_{vol}$ an Makroporen zumindest 0,35 beträgt, wobei $p_{vol}$ bestimmt ist durch

$$p_{vol} = \frac{V_{0,26-2}}{V_{0,02-6,5}}$$

worin

$V_{0,26-2}$ für das Volumen der Poren mit mittleren Durchmessern im Bereich von 0,26 bis 2 $\mu$m steht, und $V_{0,02-6,5}$ für das Volumen der Poren mit mittleren Durchmessern im Bereich von 0,02 bis 6,5 $\mu$m steht, wobei das Volumen der Poren durch Quecksilberporosimetrie bestimmt wird.

3. Katalysator nach einem der vorhergehenden Ansprüche, **dadurch gekennzeichnet, dass** der Trägerformkörper ein hohlzylindrischer Trägerformkörper ist.

4. Katalysator nach einem der vorhergehenden Ansprüche, **dadurch gekennzeichnet, dass** der Trägerformkörper aus Steatit besteht und im wesentlichen unporös ist.

5. Katalysator nach Anspruch 3 oder 4, **dadurch gekennzeichnet, dass** der hohlzylindrische Trägerformkörper eine Höhe von 2 bis 5 mm und einen Außendurchmesser von 4 bis 8 mm aufweist und der Halbwert der Differenz des Außendurchmessers und Innendurchmessers 1 bis 2 mm beträgt.

6. Katalysator nach einem der vorhergehenden Ansprüche, **dadurch gekennzeichnet, dass** die Aktivmasse ein Multielementoxid der allgemeinen Formel (II) umfasst

$$Mo_{12}V_aW_bCu_cX^4_eX^5_fO_n \qquad (II)$$

worin

X⁴ für eines oder mehrere Alkali- und/oder Erdalkalimetalle steht,
$X^5$ für eines oder mehrere Elemente aus der Gruppe Si, Al, Ti und Zr steht,
a für eine Zahl im Bereich von 2 bis 4 steht,
b für eine Zahl im Bereich von 0 bis 3 steht,
c für eine Zahl im Bereich von 0,5 bis 3 steht,
e für eine Zahl im Bereich von 0 bis 4 steht,
f für eine Zahl im Bereich von 0 bis 40 steht, und
n für den stöchiometrischen Koeffizienten des Elements Sauerstoff steht, der durch die stöchiometrischen Koeffizienten der von Sauerstoff verschiedenen Elemente sowie deren Ladungszahl in (II) bestimmt wird.

7. Verfahren zur Herstellung des Katalysators nach einem der vorhergehenden Ansprüche, wobei man den Trägerformkörper mit der Aktivmasse beschichtet, indem man eine Vielzahl von Trägerformkörpern, eine pulverförmige Aktivmasse und ein flüssiges Bindemittel, ohne die pulverförmige Aktivmasse mit dem flüssigen Bindemittel zu sättigen, in einem Behälter durchmischt, **dadurch gekennzeichnet, dass** man die Trägerformkörper in dem Behälter vorlegt und die pulverförmige Aktivmasse und das flüssige Bindemittel getrennt voneinander über die Dauer der Beschichtung hinweg in den Behälter gibt und die Dauer des Beschichtungsvorgangs weniger als 30 Minuten beträgt.

8. Verfahren nach Anspruch 6 oder 7, **dadurch gekennzeichnet, dass** das Durchmischen durch kontinuierliche Bewegung des Behälters erfolgt.

9. Verfahren nach Anspruch 8, **dadurch gekennzeichnet, dass** die Bewegung eine Rotationsbewegung ist.

10. Verfahren nach einem der Ansprüche 6 bis 9, **dadurch gekennzeichnet, dass** die pulverförmige Aktivmasse einen numerischen Anteil von Partikeln mit einer Längstausdehnung oberhalb von 50 $\mu$m von weniger als 1 % aufweist.

11. Verfahren zur Herstellung einer $\alpha,\beta$-ungesättigten Carbonsäure durch Gasphasenoxidation eines $\alpha,\beta$-ungesättigten Aldehyds mit molekularem Sauerstoff an einem Katalysatorfestbett, **dadurch gekennzeichnet, dass** das Katalysatorfestbett eine Schüttung eines Katalysators nach einem der Ansprüche 1 bis 6 umfasst.

12. Verfahren nach Anspruch 11, **dadurch gekennzeichnet, dass** das Katalysatorfestbett zumindest zwei aufeinanderfolgende Reaktionszonen umfasst und die Schüttung zumindest in der zum Reaktoreingang nächsten Reaktionszone einen Katalysator nach einem der Ansprüche 1 bis 6 umfasst.

13. Verfahren nach Anspruch 11 oder 12, wobei das Katalysatorfestbett zumindest zwei aufeinanderfolgende Reaktionszonen umfasst und die volumenspezifische Katalysatoraktivität vom Reaktoreingang zum Reaktorausgang von einer Reaktionszone zur nächsten zunimmt, **dadurch gekennzeichnet, dass** die Schüttung zumindest in der Reaktionszone, in der die höchste lokale Temperatur auftritt, einen Katalysator nach einem der Ansprüche 1 bis 6 umfasst.

14. Verfahren nach Anspruch 11 bis 13, **dadurch gekennzeichnet, dass** man ab einem bestimmten Betriebszeitpunkt zur Rückgewinnung der Qualität der Schüttung nicht die gesamte gebrauchte Schüttung sondern nur die Teilmenge der Schüttung, in der die höchste lokale Temperatur auftritt entnimmt und durch eine frische Schüttung ersetzt.

15. Verfahren nach Anspruch 11 bis 14 zur Herstellung von Acrylsäure durch Gasphasenoxidation von Acrolein.

## Claims

1. A catalyst for preparation of an $\alpha,\beta$-unsaturated carboxylic acid by gas phase oxidation of an $\alpha,\beta$-unsaturated aldehyde, comprising a shaped support body with an active composition applied thereto, wherein the active composition coverage q

$$q = \frac{Q}{(100-Q)\,S_m}$$

is at most 0.22 mg/mm$^2$, where Q is the active composition content of the catalyst in % by weight and $S_m$ is the specific geometric surface area of the shaped support body in mm$^2$/mg and the active composition comprises a catalytically active multielement oxide composition comprising the elements Mo and V, where the molar proportion of the element Mo in the total amount of all elements other than oxygen in the catalytically active multielement oxide composition is 20 mol% to 80 mol%, the molar ratio of Mo present in the catalytically active multielement oxide composition to V present in the catalytically active multielement oxide composition, Mo/V, is 15:1 to 1:1.

2. The catalyst according to claim 1, wherein the proportion by volume $p_{vol}$ of macropores is at least 0.35, where $p_{vol}$ is determined by

$$p_{vol} = \frac{V_{0.26-2}}{V_{0.02-6.5}}$$

in which

$V_{0.26-2}$ is the volume of the pores having mean diameters in the range from 0.26 to 2 $\mu$m, and
$V_{0.02-6.5}$ is the volume of the pores having mean diameters in the range from 0.02 to 6.5 $\mu$m, where the volume of the pores is determined by mercury porosimetry.

3. The catalyst according to either of the preceding claims, wherein the shaped support body is a hollow cylindrical shaped support body.

4. The catalyst according to either of the preceding claims, wherein the shaped support body consists of steatite and is essentially nonporous.

5. The catalyst according to claim 3 or 4, wherein the hollow cylindrical shaped support body has a height of 2 to 5 mm and an external diameter of 4 to 8 mm, and the median difference between the external diameter and internal diameter is 1 to 2 mm.

6. The catalyst according to either of the preceding claims, wherein the active composition comprises a multielement oxide of the general formula (II)

$$Mo_{12}V_aW_bCu_cX^4{}_eX^5{}_fO_n \qquad (II)$$

in which

$X^4$ is one or more alkali metals and/or alkaline earth metals,
$X^5$ is one or more elements from the group of Si, Al, Ti and Zr,
a is a number in the range from 2 to 4,
b is a number in the range from 0 to 3,
c is a number in the range from 0.5 to 3,
e is a number in the range from 0 to 4,
f is a number in the range from 0 to 40, and
n is the stoichiometric coefficient of the element oxygen, which is determined by the stoichiometric coefficients of the elements other than oxygen and the valency thereof in (II) .

7. A process for preparing the catalyst according to any of the preceding claims, by coating the shaped support body with the active composition, by mixing a multitude of shaped support bodies, a pulverulent active composition and a liquid binder, without saturating the pulverulent active composition with the liquid binder, in a vessel, wherein the shaped support bodies are initially charged in the vessel and the pulverulent active composition and the liquid binder are added to the vessel separately from one another over the duration of the coating and the duration of the coating operation is less than 30 minutes.

8. The process according to claim 6 or 7, wherein the mixing is effected by continuous movement of the vessel.

9. The process according to claim 8, wherein the movement is a rotational movement.

10. The process according to any of claims 6 to 9, wherein the pulverulent active composition has a numerical proportion of particles having a longest dimension above 50 $\mu$m of less than 1%.

11. A process for preparing an $\alpha,\beta$-unsaturated carboxylic acid by gas phase oxidation of an $\alpha,\beta$-unsaturated aldehyde with molecular oxygen over a fixed catalyst bed, wherein the fixed catalyst bed comprises a bed of a catalyst according to any of claims 1 to 6.

12. The process according to claim 11, wherein the fixed catalyst bed comprises at least two successive reaction zones and the bed, at least in the reaction zone closest to the reactor inlet, comprises a catalyst according to any of claims 1 to 6.

13. The process according to claim 11 or 12, wherein the fixed catalyst bed comprises at least two successive reaction zones and the volume-specific catalyst activity increases from one reaction zone to the next from the reactor inlet to the reactor outlet,
wherein the bed, at least in the reaction zone in which the highest local temperature occurs, comprises a catalyst according to any of claims 1 to 6.

14. The process according to claim 11 to 13, wherein, from a certain operating time, to restore the quality of the bed, not the entire spent bed but only the portion of the bed in which the highest local temperature occurs is removed and replaced by a fresh bed.

15. The process according to claim 11 to 14 for preparation of acrylic acid by gas phase oxidation of acrolein.

**Revendications**

1. Catalyseur pour la fabrication d'un acide carboxylique $\alpha,\beta$-insaturé par oxydation en phase gazeuse d'un aldéhyde $\alpha,\beta$-insaturé, comprenant un corps moulé support sur lequel est appliquée une masse active, **caractérisé en ce que** le recouvrement de masse active q

$$q = \frac{Q}{(100-Q)\,S_m}$$

est d'au plus 0,22 mg/mm$^2$, Q étant la proportion de masse active du catalyseur en % en poids et $S_m$ étant la surface géométrique spécifique du corps moulé support en mm$^2$/g, et la masse active comprenant une masse d'oxyde de plusieurs éléments catalytiquement active contenant les éléments Mo et V, la proportion molaire de l'élément Mo par rapport à la quantité totale de tous les éléments différents de l'oxygène de la masse d'oxyde de plusieurs éléments catalytiquement active étant de 20 % en moles à 80 % en moles, et le rapport molaire entre le Mo contenu dans la masse d'oxyde de plusieurs éléments catalytiquement active et le V contenu dans la masse d'oxyde de plusieurs éléments catalytiquement active, Mo/V, étant de 15:1 à 1:1.

2. Catalyseur selon la revendication 1, **caractérisé en ce que** la proportion en volume $p_{vol}$ de macropores est d'au moins 0,35, $p_{vol}$ étant déterminé par :

$$p_{vol} = \frac{V_{0,26-2}}{V_{0,02-6,5}}$$

Dans laquelle

$V_{0,26-2}$ représentant le volume des pores ayant des diamètres moyens dans la plage allant de 0,26 à 2 $\mu$m, et $V_{0,02-6,5}$ représentant le volume des pores ayant des diamètres moyens dans la plage allant de 0,02 à 6,5 $\mu$m, le volume des pores étant déterminé par porosimétrie au mercure.

3. Catalyseur selon l'une quelconque des revendications précédentes, **caractérisé en ce que** le corps moulé support est un corps moulé support cylindrique creux.

4. Catalyseur selon l'une quelconque des revendications précédentes, **caractérisé en ce que** le corps moulé support est constitué par de la stéatite et est essentiellement non poreux.

5. Catalyseur selon la revendication 3 ou 4, **caractérisé en ce que** le corps moulé support cylindrique creux présente une hauteur de 2 à 5 mm et un diamètre extérieur de 4 à 8 mm, et la demi-valeur de la différence entre le diamètre extérieur et le diamètre intérieur est de 1 à 2 mm.

6. Catalyseur selon l'une quelconque des revendications précédentes, **caractérisé en ce que** la masse active comprend un oxyde de plusieurs éléments de formule générale (II) :

$$Mo_{12}V_aW_bCu_cX^4_eX^5_fO_n \qquad (II)$$

dans laquelle

$X^4$ représente un ou plusieurs métaux alcalins et/ou alcalino-terreux,
$X^5$ représente un ou plusieurs éléments du groupe constitué par Si, Al, Ti et Zr,
a représente un nombre dans la plage allant de 2 à 4,
b représente un nombre dans la plage allant de 0 à 3,
c représente un nombre dans la plage allant de 0,5 à 3,
e représente un nombre dans la plage allant de 0 à 4,
f représente un nombre dans la plage allant de 0 à 40, et
n représente le coefficient stoechiométrique de l'élément oxygène, qui est déterminé par les coefficients stoechiométriques des éléments différents de l'oxygène et leur valence dans (II).

7. Procédé de fabrication du catalyseur selon l'une quelconque des revendications précédentes, selon lequel le corps moulé support est revêtu avec la masse active par mélange d'une pluralité de corps moulés supports, d'une masse active en poudre et d'un liant liquide, sans saturer la masse active en poudre avec le liant liquide, dans un contenant, **caractérisé en ce que** les corps moulés supports sont chargés initialement dans le contenant, et la masse active en poudre et le liant liquide sont ajoutés séparément l'un de l'autre pendant la durée du revêtement dans le contenant, et la durée du processus de revêtement est inférieure à 30 minutes.

8. Procédé selon la revendication 6 ou 7, **caractérisé en ce que** le mélange a lieu par un mouvement continu du contenant.

9. Procédé selon la revendication 8, **caractérisé en ce que** le mouvement est un mouvement de rotation.

10. Procédé selon l'une quelconque des revendications 6 à 9, **caractérisé en ce que** la masse active en poudre présente une proportion numérique de particules ayant une dimension longitudinale supérieure à 50 $\mu$m de moins de 1 %.

11. Procédé de fabrication d'un acide carboxylique $\alpha,\beta$-insaturé par oxydation en phase gazeuse d'un aldéhyde $\alpha,\beta$-insaturé avec de l'oxygène moléculaire sur un lit catalytique fixe, **caractérisé en ce que** le lit catalytique fixe comprend un garnissage d'un catalyseur selon l'une quelconque des revendications 1 à 6.

12. Procédé selon la revendication 11, **caractérisé en ce que** le lit catalytique fixe comprend au moins deux zones de réaction successives, et le garnissage au moins dans la zone de réaction la plus proche de l'entrée du réacteur comprend un catalyseur selon l'une quelconque des revendications 1 à 6.

13. Procédé selon la revendication 11 ou 12, selon lequel le lit catalytique fixe comprend au moins deux zones de réaction successives, et l'activité catalytique spécifique au volume augmente depuis l'entrée du réacteur vers la sortie du réacteur d'une zone de réaction à la suivante, **caractérisé en ce que** le garnissage comprend, au moins dans la zone de réaction dans laquelle la température locale la plus élevée se produit, un catalyseur selon l'une quelconque des revendications 1 à 6.

14. Procédé selon les revendications 11 à 13, **caractérisé en ce qu'**à partir d'un moment donné de l'exploitation, pour récupérer la qualité du garnissage, seule la partie du garnissage dans laquelle la température locale la plus élevée se produit, et non la totalité du garnissage usagé, est extraite et remplacée par un garnissage frais.

15. Procédé selon les revendications 11 à 14, pour la fabrication d'acide acrylique par oxydation en phase gazeuse d'acroléine.

# FIG.1a

FIG.1b

FIG.2

# FIG.3

FIG.4

FIG.5

FIG.6

FIG.7

# FIG.8

FIG.9

FIG.10

## FIG.11

FIG.12

## FIG.13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 714700 A
- DE 19927624 A
- DE 10360057 A
- WO 2011134932 A1
- DE 10028582 A
- WO 2005030380 A2
- WO 2004085370 A
- WO 2011134932 A
- DE 102007010422
- WO 9511081 A
- DE 2909671 A
- DE 102005010645 A
- DE 2830765 C

- DE 2513405 C
- US 3147084 A
- DE 2201528 A
- EP 383224 A
- DE 2903582 A
- EP 873783 A
- DE 10360057 A1
- DE 102007010422 A1
- DE 2135620 A
- DE 10232748
- EP 1525178 A0
- DE 201010048405

**In der Beschreibung aufgeführte Nicht-Patentliteratur**

- **GEORGES REBER.** Eigenschaften und Einsatzmöglichkeiten von Aerogelfenstern im Vergleich mit konventionellen sowie evakuierten Fenstern. *Inauguraldissertation,* 1991